# FASCICULE DE BREVET EUROPEEN

(11) **EP 2 688 574 B1**
(45) Date de publication et mention de la délivrance du brevet: **14.08.2019**
(21) Numéro de dépôt: 12711154.0
(22) Date de dépôt: 23.03.2012
(51) Int. Cl.: A61K 31/7105, A61K 39/395, A61K 39/21, G01N 33/50, C12Q 1/44, C12Q 1/70

(54) **MÉTHODE DE CRIBLAGE DE COMPOSÉS ANTIRÉTROVIRAUX ET VACCIN**
SCREENING-VERFAHREN ZUR AUFFINDUNG ANTIRETROVIRALEN VERBINDUNGEN UND IMPFSTOFF
METHOD FOR SCREENING ANTIRETROVIRAL COMPOUNDS AND VACCINE

(30) Priorité: 23.03.2011 FR 1152394
(43) Date de publication de la demande: 29.01.2014
(73) Titulaire: Centre National de la Recherche Scientifique (C.N.R.S.), 75016 Paris (FR)
(72) Inventeur: BENKIRANE, Monsef, F-34980 Saint Gely du Fesc (FR); LAGUETTE, Nadine, 34680 Saint Georges d'Orgues (FR); SOBHIAN, Bijan, 34680 Saint Georges d'Orgues (FR)
(74) Mandataire: Lavoix
(86) Numéro de dépôt international: PCT/EP2012/055237
(87) Numéro de publication internationale: WO 2012/127041

(56) Documents cités:
- WO-A1-2010/028264
- WO-A2-2005/081911
- WO-A2-2010/060967
- US-A- 6 043 081
- US-A1- 2004 234 956
- LAGUETTE N. ET AL.: "SAMHD1 is the dendritic- and myeloid-cell-specific HIV-1 restriction factor counteracted by Vpx", NATURE, vol. 474, no. 7353, 30 juin 2011 (2011-06-30), pages 654-657, XP055020127,
- HRECKA K. ET AL.: "Vpx relieves inhibition of HIV-1 infection of macrophages mediated by the SAMHD1 protein", NATURE, vol. 474, no. 7353, 30 juin 2011 (2011-06-30), pages 658-661, XP055020191,
- LIM E.S. ET AL.: "HIV: Going for the watchman", NATURE, vol. 474, no. 7353, 30 juin 2011 (2011-06-30), pages 587-588, XP055020313,
- RICE G.I. ET AL.: "Mutations involved in Aicardi-Goutières syndrome implicate SAMHD1 as regulator of the innate immune response", NATURE GENETICS, vol. 41, no. 7, juillet 2009 (2009-07), pages 829-832, XP055020329,

## Description

Le virus de l'immunodéficience humaine (VIH) est l'agent causal du syndrome d'immunodéficience acquise (SIDA), une maladie infectieuse représentant l'un des problèmes majeurs de santé publique auquel est confrontée la médecine moderne.

Selon des chiffres publiés par l'OMS en 2008, 33,4 millions de personnes vivent avec le VIH. On estime actuellement à 2,7 millions le nombre de primo-infections par le virus et à 2 millions le nombre de décès entraînés par le sida. L'Afrique subsaharienne est la région la plus touchée réunissant 67% des cas mondiaux, 68% des nouveaux cas enregistrés chez les adultes et 91% chez les enfants.

Les principales cellules cibles du VIH sont des cellules du système immunitaire, en particulier les lymphocytes T CD4+, mais également les cellules dendritiques, les monocytes et les macrophages. Dans la mesure où le VIH interfère avec les fonctions immunitaires des cellules cibles, et que sa réplication provoque la destruction de ces cellules, l'infection conduit à long terme à un affaiblissement du système immunitaire rendant l'organisme vulnérable aux maladies opportunistes. Ainsi, en l'absence de traitement antirétroviral, la quasi-totalité des sujets infectés par le VIH évolue vers le SIDA, ultime expression clinique de la destruction progressive du système immunitaire.

A partir de 1996, l'introduction de multithérapies mettant en oeuvre des inhibiteurs de protéases et des inhibiteurs de la transcriptase inverse a abouti à une réduction massive de la morbidité et des infections opportunistes, retardant ainsi la progression de l'infection vers un stade SIDA. Cependant, à ce jour, il n'existe pas de traitement permettant d'éradiquer le virus de l'organisme et de guérir définitivement de l'infection par le VIH.

En outre, malgré des recherches intensives menées depuis plus de vingt ans dans le domaine de la vaccinologie, une stratégie vaccinale curative ou préventive efficace n'a pas encore pu être identifiée.

Le virus de l'immunodéficience humaine appartient à la famille des *Retroviridae* et au genre Lentivirus. En ce qui concerne les lentivirus de primates, l'analyse phylogénique comparative des VIH et des virus simiens VIS a révélé la proximité génétique entre les VIH-1, VIS de chimpanzé (VIScpz) et le VIS de gorille (VISgor) d'une part, et entre le VIH-2 et le VIS de Mangabey (VISsm) d'autre part, ce qui consolide l'hypothèse d'une origine simienne des VIH-1 et VIH-2.

Les virus du genre Lentivirus se différencient des autres genres de rétrovirus par leur capacité à se répliquer aussi bien dans des cellules en division que dans des cellules qui ne se divisent pas, cette caractéristique faisant de ces virus de bons candidats pour le développement de vecteurs lentiviraux utilisables dans le cadre de thérapies géniques.

Le génome des VIH et des VIS est constitué de deux brins d'ARN simple brin homologues de polarité positive comprenant neuf gènes. Les trois principaux gènes rétroviraux, communs à tous les rétrovirus, sont les gènes *gag, pol* et *env*, qui codent des polyprotéines qui une fois maturées par la protéase virale produisent les protéines structurales (protéine de matrice, protéine de capside, protéine de nucléocapside, protéines d'enveloppe gp41 et gp120), et les enzymes virales impliquées dans la réplication du virus, dans son intégration dans le génome de la cellule hôte et dans la maturation des polyprotéines précurseurs (respectivement la transcriptase inverse, l'intégrase et la protéase). Deux autres gènes rétroviraux codent les protéines régulatrices tat et rev : la protéine tat est impliquée dans la régulation de la transcription des gènes viraux suite à l'intégration de l'ADN rétroviral dans le génome cellulaire et de gènes cellulaires, alors que rev participe à l'export nucléaire des transcrits.

Quatre autres gènes nommés *nef*, *vif*, *vpr* et *vpu* codent des protéines dites accessoires agissant sur différentes étapes du cycle viral et sur la régulation de différentes voies cellulaires. Dans le cas des VIH-2 et de certains VIS, notamment VISsm, le gène *vpu* codant la protéine Vpu est absent et est remplacé par le gène *vpx* codant une protéine accessoire nommée Vpx.

Les virions VIH/VIS matures ont une morphologie sphérique de 80 à 120 nm de diamètre et sont constitués d'une l'enveloppe virale (une membrane phospholipidique dans laquelle sont enchâssées les spicules constituées de trois glycoprotéines transmembranaires gp41 liées de manière covalente à un trimère de la glycoprotéine gp120 (Lu et al., Nature Structural Biology, 2(12): 1075-1082, 1995)) qui entoure la matrice et une nucléocapside de forme conique. La nucléocapside renferme le génome viral, la protéase, la transcriptase inverse, l'intégrase, ainsi que quelques protéines virales Vif, Vpr, Nef et Vpu (ou Vpx dans le cas de VIH-2 et certains VIS, notamment VISsm).

Le cycle du VIH comporte les étapes suivantes :
- fixation du virus à la membrane plasmique de la cellule cible par l'intermédiaire d'une interaction spécifique entre la glycoprotéine virale gp120 et la molécule de surface cellulaire CD4 ;
- changement conformationnel de gp120 et liaison avec un corécepteur présent près de CD4, à savoir le récepteur de chimiokine CCR5 ou CXCR4 ;
- fusion entre l'enveloppe virale et la membrane cytoplasmique par l'intermédiaire de gp41 ;
- pénétration du virus dans la cellule et décapsidation de la particule virale ;
- transcription inverse de l'ARN viral génomique en ADN double brin via la transcriptase inverse présente dans la particule virale (structure compatible avec celle de l'ADN cellulaire dans lequel le l'ADN viral sera intégré afin d'assurer la réplication du virus et son expression) ;
- importation de l'ADN double brin viral néoformé dans le noyau cellulaire, puis intégration dans le génome cellulaire via l'intégrase virale (l'ADN viral est alors appelé ADN proviral) ;
- expression de l'ADN proviral, formation et libération de nouvelles particules virales

Chacune des étapes du cycle viral est une cible potentielle pour la mise au point de composés antirétroviraux. C'est ainsi que les premiers antirétroviraux anti-VIH développés étaient des inhibiteurs de la protéase rétrovirale et des inhibiteurs de la transcriptase inverse. Plus récemment, des molécules inhibitrices de la fusion entre l'enveloppe virale et la membrane cellulaire ont été mises au point.

Dans le cadre de la recherche de molécules capables de bloquer l'infection par le VIH, ces dernières années ont été marquées par la découverte de facteurs cellulaires chez les primates, connus sous le nom de facteurs de restriction, qui interfèrent avec le cycle de réplication de certains rétrovirus dont VIH/VIS. Ces facteurs de restriction sont capables de limiter la réplication des VIH/VIS après leur entrée dans la cellule hôte.

Le premier facteur de restriction ayant une activité contre le VIH, à savoir la protéine TRIM5 alpha, a été mis en évidence en 2004 (Stremlau et al., Nature, 427 : 848-853, 2004). Stremlau et ses collaborateurs cherchant à identifier les gènes à l'origine de la résistance de certains primates non humains au VIH-1, notamment le macaque rhésus, ont criblé une banque d'ADNc de cellules de singe rhésus, et ont découvert que la protéine TRIM5 alpha simienne, une fois exprimée par des cellules humaines, protégeait ces cellules contre VIH-1 mais pas contre l'homologue simien. La protéine TRIM5 alpha simienne n'empêche pas le VIH-1 de pénétrer dans les cellules, mais elle bloque très rapidement le cycle viral.

Des études ultérieures ont montré que la spécificité de restriction du VIH-1 par les protéines TRIM5 alpha d'origine simienne est portée par la proline en position 332 du domaine nommé SPRY localisé dans la partie C-terminale de la protéine. La simple mutation R332P dans le domaine SPRY de son homologue humain confère à ce dernier la capacité d'inhiber VIH-1.

Récemment, l'existence d'un autre facteur de restriction pour le VIH a été mise en évidence. Ainsi, il a été montré que les cellules dendritiques et les cellules de la lignée monocytaire, notamment les macrophages, expriment un facteur de restriction capable de limiter leur infection par certains lentivirus, en particulier le VIH-1 et certains VIS. Il a également été montré que la protéine auxiliaire Vpx exprimée par le VIH-2 et par certains VIS, notamment VISsm, est capable de contrecarrer l'action de ce facteur de restriction, ceci expliquant que ce facteur de restriction est inefficace contre le VIH-2 et que VIH-2 infecte de façon efficace les cellules dendritiques dès le début de l'infection.

De nombreuses études ont été menées afin de caractériser davantage le mécanisme d'action de ce facteur de restriction et d'identifier le facteur cellulaire qui en est à l'origine (pour revue Ayinde et al., Retrovirology, 7:35, 2010).

Bien que le facteur de restriction contrecarré par Vpx n'ait pas été identifié, il a été démontré que ce facteur de restriction est exprimé de façon constitutive dans les cellules. Ce facteur de restriction ne prévient pas la pénétration du virus dans la cellule mais semble empêcher l'accumulation d'ADN issu de la transcription inverse de l'ARN génomique, étape du cycle viral également ciblée par le facteur de restriction TRIM5 alpha. Par ailleurs, comme TRIM5 alpha, le facteur inhibé par Vpx semble inductible par les interférons de type 1 (Gramberg et al., Curr HIV/AIDS Rep., 6: 36-42, 2009). Cependant, ce nouveau facteur de restriction diffère de TRIM5 alpha par plusieurs aspects. Alors que l'expression de TRIM5 alpha est ubiquitaire, le facteur de restriction inhibé par Vpx est exprimé uniquement dans les cellules dendritiques et les cellules de la lignée monocytaire. De plus, contrairement à TRIM5 alpha, ce facteur de restriction semble ne pas être saturable puisque un large excès de particules virales ne permet pas de restaurer l'infectivité d'un VIS de macaque qui n'exprime pas une protéine Vpx fonctionnelle (Goujon et al., Retrovirology, 4:2, 2007).

Le facteur de restriction inhibé par Vpx étant spécifiquement exprimé par les cellules dendritiques et les cellules de la lignée monocytaire, des travaux récents se sont intéressés à l'implication de ce facteur de restriction dans la réponse immunitaire innée médiée par ces cellules. Lors d'une infection, les cellules dendritiques jouent un rôle essentiel dans la réponse innée, notamment par expression et sécrétion d'interféron de type 1. Ces cellules sont également spécialisées dans la capture, l'apprêtement et la présentation d'antigènes, et jouent un rôle central dans la réponse immunitaire car ce sont les seules capables d'activer les lymphocytes T naïfs et de déclencher une réponse immune primaire coordonnée et durable du système immunitaire, permettant la protection de l'organisme contre l'agent infectieux. Or, dans le cas d'une infection par VIH par voie sexuelle, en particulier VIH-1, la réponse immunitaire innée est faible durant la phase aiguë (c'est-à-dire la première semaine suivant l'infection), les interférons de type 1 (qui ont une activité antivirale) ne sont en général par exprimés.

Des travaux récents ont montré que lorsque la résistante à l'infection des cellules dendritiques est contournée (co-infection par le virus VIH-1 et des pseudoparticules virales apportant Vpx), l'infection par VIH-1 induit la maturation des cellules infectées, une réponse antivirale médiée par les interférons de type 1, ainsi qu'une activation des cellules T (Manel et al., Nature, 467(9): 214-217, 2010). Il est donc suggéré que le facteur de restriction inhibé par Vpx pourrait profiter à la stratégie d'échappement de VIH-1 dans la mesure où une réplication efficace dans les cellules dendritiques induirait une réponse immunitaire innée forte, notamment par expression d'interféron de type 1, qui pourrait permettre dès les premiers jours post-infection la mise en place d'une réponse immunitaire efficace.

Ces études suggèrent que l'inefficacité des vaccins anti-VIH-1 actuels serait principalement due à la restriction de l'infection par VIH-1 dans les cellules dendritiques et les macrophages. L'identification du facteur de restriction exprimé par ces cellules est donc cruciale pour l'élaboration d'un vaccin efficace contre les rétrovirus, les lentivirus en général, préférentiellement contre les lentivirus qui n'expriment pas Vpx, et le VIH-1 en particulier.

En outre, l'identification de ce facteur de restriction est importante pour le développement de méthodes de transfert de gène utilisant des vecteurs rétroviraux, en particuliers lentiviraux. En effet, l'inhibition de ce facteur de restriction permettra d'améliorer la transduction des cellules exprimant ce facteur, en particulier les cellules dendritiques et les cellules de la lignée monocytaire, par les vecteurs lentiviraux à des fins de thérapie génique ou pour des expériences de transfert de gène *in vitro.*

Cependant, malgré les nombreuses études portant sur ce facteur de restriction, son identité restait jusqu'à présent inconnue.

Les inventeurs ont maintenant identifié ce facteur de restriction comme étant la protéine SAMHD1.

Le gène SAMHD1 (GenelD 25939), localisé sur le chromosome 20, est transcrit en un ARNm (numéro d'accession NCBI NM_015474) qui est traduit en une protéine SAMHD1 constituée de 626 résidus (numéro d'accession NCBI NP_056289 ; SEQ ID NO : 1).

La protéine SAMHD1 possède deux régions identifiables. Une première (résidus 44 à 107) nommée « SAM » (pour « Steril Alpha Motif »), et une seconde (résidus 162-335), nommée « HDc », qui présente une activité phosphohydrolase (RNase).

La protéine SAMHD1, également nommée DCPI (pour « dendritic cell-derived IFN-gamma induced protein ») a été identifiée suite au criblage d'une banque d'ADNc provenant de cellules dendritiques et s'est révélée être une protéine orthologue à la protéine murine MG11, protéine induite par les interférons gamma (Li et al., Immunology Letters, 74: 221-224, 2000). SAMHD1 pourrait être impliquée dans la réponse antivirale innée (Hartman et al., J. Virol., 81: 1796-1812, 2007), et jouer un rôle dans la réponse proinflammatoire induite par le TNF alpha (Lio et al., Proteomics, 8: 2640-2650, 2008). Récemment, il a été démontré que des mutations dans le gène codant SAMHD1 étaient associées au syndrome de Aicardi-Goutières, une maladie génétique dont les symptômes sont proches de ceux rencontrés lors d'une infection virale congénitale (Rice et al., Nature Genetics, 41(7): 829-832, 2009 ; Dale et al., Am. J. Med. Genet. A., 152A(4): 938-942, 2010 ; Thiele et al., Hum. Mutat., 31(11): 1836-50, 2010). Il a également été proposé que SAMHD1 joue un rôle protecteur afin d'éviter une auto-activation de la réponse immunitaire innée (Rice et al., Nature Genetics, 41(7): 829-832, 2009). Cependant, le lien entre la résistance à l'infection par VIH-1 des cellules dendritiques et des cellules de la lignée monocytaire et l'expression de SAMHD1 n'avait jamais été évoqué.

Les inventeurs ont en outre recherché la ou les régions impliquée(s) dans la restriction. Ils ont ainsi montré que l'activité phosphohydrolase de cette protéine est requise pour la restriction de l'infection par VIH-1. Des cellules dendritiques exprimant une protéine SAMHD1 mutée dans le domaine « HDc » ne sont plus résistantes à l'infection, le virus VIH-1 se répliquant fortement dans ces cellules.

### METHODE IN VITRO DE CRIBLAGE

Les inventeurs ayant identifié la protéine SAMHD1 comme le facteur de restriction inhibant la réplication de VIH-1, l'invention a comme objet une méthode *in vitro* de criblage de composés candidats susceptibles d'être utilisés pour le traitement préventif et/ou curatif d'une maladie causée par un rétrovirus, telle que définie dans les revendications.

Cette méthode comprend les étapes suivantes :
- mettre en contact un composé candidat avec une cellule exprimant SAMHD1 ou avec la protéine SAMHD1 ; et
- déterminer si le composé candidat module l'expression et/ou l'activité de la protéine SAMHD1 ;
dans laquelle ledit composé candidat qui module l'expression et/ou l'activité de la protéine SAMHD1 est identifié comme composé candidat susceptible d'être utilisé pour le traitement préventif et/ou curatif d'une maladie causée par un rétrovirus.

Le rétrovirus qui cause la maladie est un lentivirus sélectionné dans le groupe constitué par:
- les lentivirus de primates (les virus de l'immunodéficience humaine, type 1 (VIH-1), les virus de l'immunodéficience humaine, type 2 (VIH-2), et les virus de l'immunodéficience simienne (VIS)).

Plus préférentiellement, le rétrovirus causant la maladie est un lentivirus de primates choisi parmi les VIH-1, les VIH-2, et les VIS. Avantageusement, il s'agit d'un VIH-1.

On entend par « moduler l'expression de la protéine SAMHD1 » soit l'augmentation soit l'inhibition de l'expression de cette protéine. L'expression de la protéine SAMHD1 peut être modulée en modulant la transcription de l'ARNm à partir du gène codant SAMHD1 et/ou la traduction de l'ARNm en protéine SAMHD1 (SEQ ID NO : 1) à partir de l'ARNm. De même, le terme « moduler l'activité de la protéine SAMHD1 » signifie amplifier ou inhiber l'activité de la protéine SAMHD1.

L'expression et l'activité de la protéine SAMHD1 peuvent être déterminées dans des cellules exprimant naturellement cette protéine, en particulier des cellules dendritiques et des cellules de la lignée monocytaire (notamment monocytes et macrophages), ou bien dans des cellules qui n'expriment pas naturellement SAMHD1 mais dans lesquelles le gène ou l'ADNc de SAMHD1 (SEQ ID NO : 2) a été introduit par génie génétique de sorte qu'il s'exprime dans cette cellule.

Les techniques pour introduire une séquence polynucléotidique dans une cellule, ainsi que les vecteurs utilisés pour la mise en oeuvre de ces techniques sont bien connus de l'homme du métier, et sont par exemple décrites par Sambrook et al. (Molecular Cloning, A Laboratory Manual, Third Ed., Cold Spring Harbor Laboratory Press, Cold Spring Harbor, N.Y., 2001). On citera par exemple la transfection des cellules par un vecteur d'expression comprenant le gène ou l'ADNc de SAMHD1, notamment les techniques utilisant la coprécipitation du vecteur d'expression avec le phosphate de calcium ou l'utilisation de complexes variés du vecteur d'expression avec des vésicules lipidiques (liposomes). Les cellules peuvent être des cellules eucaryotes ou des cellules procaryotes.

Les méthodes pour déterminer l'expression d'une protéine dans une cellule sont bien connues de l'homme du métier. L'expression de SAMHD1 pourra par exemple être déterminée en quantifiant la protéine SAMHD1 présente dans le lysat cellulaire, notamment à l'aide d'anticorps spécifiques de cette protéine, de techniques de chromatographie ou de techniques de spectrométrie de masse. L'expression de SAMHD1 peut également être déterminée en quantifiant l'ARNm de SAMHD1, notamment par RT-PCR quantitative en utilisant des amorces s'hybridant spécifiquement à cette ARNm.

En outre, puisque l'activité phosphohydrolase est indispensable à l'activité de la protéine SAMHD1, l'activité de cette protéine pourra être évaluée *in vitro* en mesurant la dégradation du substrat de SAMHD1, c'est-à-dire l'ARN rétoviral et/ou l'ADN rétroviral (obtenu par transcription inverse), apporté en quantité connue. L'effet du composé candidat sur l'activité de SAMHD1 sera donc évalué en mesurant le substrat restant et/ou le produit de dégradation, et en comparant cette ou ces mesures avec celles obtenues en l'absence du composé candidat (voir par exemple Zimmerman et al., JMB, 378: 215-226, 2008).

Dans ce mode de réalisation, la protéine SAMHD1 utilisée sera préférentiellement sous forme purifiée ou partiellement purifiée. Elle pourra être produite par les techniques classiques du génie génétique, telles que celles décrites par Sambrook et al. (Molecular Cloning, A Laboratory Manual, Third Ed., Cold Spring Harbor Laboratory Press, Cold Spring Harbor, N.Y., 2001), par exemple par clonage du gène ou de l'ADNc codant la protéine SAMHD1 dans un vecteur d'expression, puis expression par une bactérie transformée par le plasmide, notamment E. *coli*, ou bien transcription puis traduction *in vitro* de SAMHD1.

Dans un mode de réalisation préféré de la méthode *in vitro* de criblage selon l'invention:
- la détermination de la capacité du composé candidat à moduler l'expression de SAMHD1 est réalisée en quantifiant l'ARNm SAMHD1 et/ou la protéine SAMHD1 ; et
- la détermination de la capacité du composé candidat à moduler l'activité de SAMHD1 est réalisée en mesurant l'activité phosphohydrolase de SAMHD1.

Lorsque le composé candidat testé via une méthode *in vitro* de criblage selon l'invention présente un effet sur l'expression et/ou l'activité de la protéine SAMHD1, il sera avantageux de s'assurer que ledit composé candidat a bien un effet sur la réplication du rétrovirus.

Pour ce faire, on pourra appliquer les étapes suivantes :
i) cultiver en présence dudit composé candidat à tester des cellules infectées par ledit rétrovirus, lesdites cellules étant choisies dans le groupe constitué par des cellules dendritiques, des monocytes et des macrophages ;
ii) mesurer la réplication du rétrovirus ;
iii) sélectionner en tant que composé candidat susceptible d'être utilisé pour le traitement préventif et/ou curatif d'une maladie causée par un rétrovirus, le composé candidat en présence duquel la réplication du rétrovirus mesurée à l'étape ii) est différente de celles obtenues dans le cas où le même rétrovirus est cultivé dans les mêmes conditions que dans l'étape i) mais en l'absence du composé candidat à tester.

Dans le cadre de la présente invention, on entend par « gène codant la protéine SAMHD1 » le gène humain codant la protéine SEQ ID NO: 1, ainsi que les gènes homologues et orthologues et les variants alléliques du gène codant la protéine SEQ ID NO : 1. De préférence, les gènes homologues et orthologues et les variants alléliques codent une protéine possédant les deux régions « SAM » et « HDc » et présentant au moins 75%, de préférence au moins 85%, plus préférentiellement au moins 90%, 92%, 95%, 98%, 99% ou 100% d'identité avec la séquence SEQ ID NO :1.

De même, le terme « protéine SAMHD1 » couvre la protéine SAMHD1 correspondant à SEQ ID NO : 1 et au numéro d'accession NCBI NP_056289, ainsi que toute protéine homologue possédant les deux régions « SAM » et « HDc » et présentant au moins 75%, de préférence au moins 85%, plus préférentiellement au moins 90%, 92%, 95%, 98%, 99% ou 100% d'identité avec la séquence SEQ ID NO :1.

Les pourcentages d'identité auxquels il est fait référence dans le cadre de l'exposé de la présente invention sont déterminés sur la base d'un alignement global des séquences à comparer, c'est-à-dire sur un alignement des séquences prises dans leur intégralité, en utilisant l'algorithme de Needleman et Wunsch (J. Mol. Biol. 48, 443-453, 1970). Cette comparaison de séquences peut être effectuée par exemple à l'aide du logiciel Needle en utilisant le paramètre « Gap open » égal à 10.0, le paramètre "Gap Extend" égal à 0.5, et une matrice « Blosum 62 ». Le logiciel Needle est par exemple disponible sur le site internet ebi.ac.uk world wide, sous la dénomination « Align ».

Dans le cadre de la présente invention, on entend par « activité de la protéine SAMHD1 » l'effet inhibiteur de SAMHD1 sur la réplication du VIH-1 (ou d'une VIS ne codant par une protéine VpX), sachant que l'activité phosphohydrolase de SAMHD1 (domaine « HDc ») est responsable de cet effet inhibiteur. Ainsi, l'effet sur l'activité de la protéine SAMHD1 peut être déterminé en mesurant l'effet du composé candidat soit sur la réplication virale, soit sur l'activité phosphohydrolase de SAMHD1.

Le « composé candidat » peut être tout type de composé.
- Dans le cas d'un composé candidat visant à inhiber l'expression du gène codant SAMHD1, il peut par exemple s'agir d'ARN interférents dirigés contre l'ARNm ou le pré-ARNm de SAMHD1, d'oligonucléotides antisens ciblant le gène codant SAMHD1, de ribozymes dirigés contre l'ARNm ou le pré-ARNm de SAMHD1.
- Dans le cas d'un composé candidat visant à inhiber l'activité de la protéine SAMHD1, il peut s'agir notamment de molécules synthétisées chimiquement (de préférence de petites molécules chimiques) ou de molécules naturelles (par exemple, extraites de plantes ou de microorganismes), d'anticorps ou de fragment d'anticorps anti-SAMHD1, ou encore d'aptamères.

Lorsqu'il s'agit de molécules synthétisées chimiquement, en particulier les petites molécules chimiques, visant à inhiber l'activité de la protéine SAMHD1, celles-ci pourront avoir été présélectionnées suite à un criblage *in silico* (ou criblage dit « virtuel »), c'est-à-dire que la structure tridimensionnelle du domaine « HDc » de SAMHD1 aura été modélisée par ordinateur, en particulier la structure du site actif de la phosphohydrolase de SAMHD1, et que les molécules auront été conçues et tester virtuellement par ordinateur de sorte qu'elles soient susceptibles d'interférer avec l'activité phosphohydrolase du domaine « HDc » (par exemple afin de bloquer le site actif).

De même, les anticorps anti-SAMHD1 testés seront de préférence dirigés contre le domaine « HDc » de SAMHD1 afin d'inhiber l'activité phosphohydrolase de SAMHD1.

### VACCIN ET COMPOSITION IMMUNOGENE

Les inventeurs ont montré que l'inhibition de l'expression de SAMHD1 conduisait à l'infection des cellules et à l'activation des cellules dendritiques, ces dernières sécrétant notamment une forte quantité d'interféron de type 1.

L'inefficacité des vaccins actuels contre le VIH-1 serait principalement due à la restriction de l'infection par VIH-1 dans les cellules dendritiques et les macrophages, cette restriction de l'infection spécifique de ces cellules empêchant le développement d'une réponse immunitaire suffisante suite à l'inoculation du vaccin.

Une composition vaccinale comprenant, en plus de la composition immunogène capable d'induire une réponse immunitaire contre un rétrovirus donnée, un composé capable d'inhiber l'expression et/ou l'activité de SAMHD1, induira une réponse immunitaire plus précoce et plus forte contre ledit rétrovirus puisque les cellules dendritiques et les cellules de la lignée monocytaire seront activées précocement.

Ainsi, un autre objet de la présente invention concerne i) une composition vaccinale ou ii) une composition immunogène, telles que définies dans les revendications, ces compositions comprenant :
- un inhibiteur de l'expression et/ou de l'activité de la protéine SAMHD1 ; et
- au moins un antigène d'un rétrovirus contre lequel on souhaite diriger la réponse immunitaire ou une séquence polynucléotidique codant cet antigène, de préférence ledit au moins un antigène est un antigène d'un lentivirus.

Les inhibiteurs de l'expression de la protéine SAMHD1 et de l'activité de la protéine SAMHD1 selon l'invention sont capables de réduire l'expression de la protéine SAMHD1, ou de l'activité de la protéine SAMHD1, d'au moins 10%, préférentiellement de 30%, plus préférentiellement d'au moins 50%, et avantageusement d'au moins 70%, 75%, 80%, 85%, 90%, 95%, ou 100%.

L'inhibiteur de l'expression et/ou de l'activité de la protéine SAMHD1 agit par interaction avec le gène SAMHD1, l'ARNm de SAMHD1, ou la protéine SAMHD1, et non de manière indirecte.

L'inhibiteur est sélectionné dans le groupe constitué par une petite molécule chimique, un ARN interférent dirigé contre l'ARNm ou le pré-ARNm de SAMHD1, un oligonucléotide antisens ciblant le gène codant SAMHD1, un ribozyme dirigé contre l'ARNm ou le pré-ARNm de SAMHD1, un anticorps anti-SAMHD1 (de préférence un anticorps humanisé), ou un dérivé d'anticorps anti-SAMHD1, et un aptamères.

Lorsque l'inhibiteur agit au niveau de la protéine, et non au niveau du gène ou du transcrit de SAMHD1, il ciblera de préférence le domaine« HDc » de SAMHD1, et avantageusement le site actif du domaine phosphohydrolase de SAMHD1 afin d'inactiver cette activité.

L'inhibiteur peut avoir été identifié par la méthode de criblage selon la présente invention.

Le terme « petite molécule » fait référence à une molécule de moins de 1000 daltons, notamment des composés organiques ou inorganiques. La conception de structure chimique assistée par ordinateur et le criblage in *silico* sont utiles dans la conception de telles molécules.

On entend ici par : « anticorps anti-SAMHD1 », une molécule d'immunoglobuline dirigée contre la protéine SAMHD1, de préférence contre le domaine « HDc » de SAMHD1 afin d'inhiber l'activité phosphohydrolase de SAMHD1 et bloquer l'activité phosphohydrolase.

Le terme « anticorps humanisé » se réfère à un anticorps produit chez un animal non humain, de préférence la souris, ayant conservé sa spécificité de liaison à la protéine SAMHD1, mais dans lequel, afin de réduire son immunogénicité chez l'homme, le plus de séquences non humaines possible ont été remplacées par les séquences humaines correspondantes. En ce qui concerne les domaines variables, les séquences remplacées sont en général les régions FR (framework), c'est-à-dire les séquences situées entre les boucles hypervariables CDRs. Diverses méthodes pour obtenir des anticorps humanisés sont bien connues en elles-mêmes (pour revue cf. par exemple ALMAGRO & FRANSSON, Frontiers in Bioscience, 13, 1619-1633, 2008).

On rappellera que les CDRs (« Complementarity Determining Régions ») d'un anticorps sont les portions des domaines variables qui sont impliquées dans la reconnaissance spécifique de l'antigène. Chaque chaîne lourde et légère d'un anticorps possède trois CDRs, dénommées VH-CDR1, VH-CDR2, VH-CDR3 dans le cas de la chaine lourde, et VL-CDR1, VL-CDR2, VL-CDR3 dans le cas de la chaîne légère.

Des dérivés d'anticorps anti-SAMHD1 peuvent être en particulier :
- tout fragment d'un anticorps anti-SAMHD1 comprenant au moins les CDR3, et de préférence comprenant en outre les CDR2 et/ou les CDR1 des chaînes lourde et légère dudit anticorps ;
- toute protéine recombinante comprenant un fragment d'anticorps anti-SAMHD1 tel que défini ci-dessus.

Des fragments d'anticorps anti-SAMHD1 utilisables conformément à l'invention sont notamment des fragments Fv, dsFv, Fab, Fab'2 ou scFv. Les fragments Fv sont constitués des domaines variables des chaînes lourde et légère VH et VL d'un anticorps, associés l'un à l'autre par des interactions hydrophobes. Le fragment dsFv est constitué d'un dimère VH :: VL relié par un pont disulfure. Les fragments scFv sont constitués des portions variables des chaînes lourdes et légères d'un anticorps, reliées entre elles par l'intermédiaire d'un lieur flexible (CLACKSON et al., Nature, 352: 624-628, 1991), formant ainsi une protéine simple-chaîne. Les fragments Fab résultent de l'action de la papaïne sur une molécule d'immunoglobuline, et contiennent chacun une chaîne légère et la première moitié d'une chaîne lourde reliées entre elles par un pont disulfure. Le fragment F(ab')2 peut être obtenu par traitement d'un anticorps par la pepsine : ce fragment comprend deux fragments Fab et une partie de la région charnière. Les fragments Fab' peuvent être obtenus à partir des fragments F(ab')2 par coupure du pont disulfure dans la région charnière.

Ces fragments se liant à l'antigène peuvent également être combinés afin d'obtenir des dérivés plurivalents, tels que les « diabodies » ou « triabodies », résultant de l'association de 2 ou 3 de ces fragments se liant à l'antigène.

Des protéines recombinantes comprenant un fragment d'anticorps anti-SAMHD1 peuvent être notamment :
- des protéines associant au moins un fragment d'anticorps anti-SAMHD1 avec au moins un fragment d'un autre anticorps; on citera à titre d'exemples, des immunoglobulines bi-spécifiques, des conjugués d'un fragment Fv ou Fab d'un anticorps anti-SAMHD1 avec un fragment Fv ou Fab d'un anticorps de spécificité différente, des « diabodies bi-spécifiques » résultant de l'association d'un fragment scFv d'un anticorps anti-SAMHD1 avec un fragment Fv ou Fab d'un anticorps de spécificité différente ;
- des protéines associant au moins un fragment d'anticorps anti-SAMHD1 avec une molécule permettant de prolonger sa demi-vie plasmatique lors de son administration in vivo, notamment avec un polypeptide hydrosoluble de masse moléculaire suffisante pour que la masse moléculaire du polypeptide de fusion ainsi obtenu soit supérieure au seuil de filtration rénale.

Des anticorps chimériques ou recombinants, des fragments scFv et leurs dérivés, etc. peuvent être obtenus par les techniques classiques du génie génétique, telles que celles décrites par Sambrook et al. (Molecular Cloning, A Laboratory Manual, Third Ed., Cold Spring Harbor Laboratory Press, Cold Spring Harbor, N.Y., 2001).

Le terme « ARN interférent » se réfère à une molécule d'ARN double-brin capable d'inhiber de manière séquence spécifique l'expression d'un gène cible en provoquant la dégradation de son ARNm.

Pour être utilisé en cellule de mammifère, les ARN interférents doivent posséder une partie double-brin de moins de 30 pb afin d'éviter une réponse interféron non-spécifique induite par des ARN double-brin plus long. Ces ARN interférents, qui sont des ARN contenant à la fois la séquence-cible et la séquence antisens correspondante, incluent en particulier les petits ARN interférents (« small interfering RNAs » ou siRNAs) (ELBASHIR et al., Nature, 411(6836): 494-8, 2001 ; TUSCHL, Nat. Biotechnol., 20(5): 446-8, 2002), les ARN courts en forme d'épingle à cheveux (« short hairpin RNAs » ou shRNAs) qui sont ensuite transformés par la machinerie cellulaire en siRNAs (PADDISON et al., Genes Dev., 16(8): 948-58, 2002; YU et al., P.N.A.S., 99(9): 6047-52, 2002; SIOLAS et al., Nat. Biotechnol., 23(2): 227-31, 2005),ainsi que les pré-miARN et les miARN.

Les siARN ont généralement une longueur de 21 à 25 nucléotides ; ils contiennent une portion double-brin, généralement de 19 à 21 nucléotides, constituée de la séquence cible et de la séquence antisens correspondante ; l'un ou l'autre brin, ou les deux, comporte(nt) généralement à l'extrémité 3' une extension simple-brin de 2 ou 3 nucléotides ; le plus souvent (mais non obligatoirement), il s'agit d'un dinucléotide, de séquence TT.

Les shARN sont constitués d'un seul brin, d'une longueur de 50 à 70 nucléotides, qui peut se replier pour former une structure en épingle à cheveux, contenant une portion double-brin d'une longueur de 19 à 29 nucléotides, constituée par la séquence cible et la séquence antisens correspondante, et une boucle de 5 à 10 nucléotides. Ils peuvent comprendre également, à l'extrémité 3', une extension simple-brin de 2 ou 3 nucléotides.

Comme indiqué précédemment, les ARN interférents peuvent aussi être utilisés sous forme de précurseurs (pré-miARN) ou de miARN. Les miARNs sont produits à partir des précurseurs pré-miARN d'environ 70 nucléotides qui présentent une forme d'épingle à cheveux comprenant une boucle. La séquence d'un pré-miARN, identifié dans les conditions naturelles dans une cellule eucaryote, peut être modifiée pour y introduire une séquence siARN, qui, libérée par DICER ira alors dégrader un autre ARNm que celui initialement ciblé. Ce pré-miARN modifié sert alors de porteur pour le siARN d'intérêt, ce qui a pour résultat d'augmenter l'efficacité de l'interférence. Avantageusement, le pré-miARN comprend, de 5' vers 3', la séquence antisens cible, suivie de la séquence de la boucle du pré-miARN naturel, puis la séquence sens cible. De préférence, la boucle est celle du pré-miARN de miR-155 provenant de l'ARN non codant BIC retrouvé notamment chez la souris, le poulet ou l'homme (KWAN-HO CHUNG et al., Nucleic Acids Research, 34(7), 2006).

Les méthodes classiques de préparation des acides nucléiques peuvent être mises en oeuvre pour produire les ARN interférents conforme à l'invention (pour revue cf. par exemple AMARZGUIOUI et al., FEBS Lett, 579, 5974, 2005). Il peut s'agir de synthèse chimique, ou bien de production par génie génétique. Dans ce dernier cas on utilisera un vecteur d'expression contenant une séquence d'ADN pouvant être transcrite en au moins un ARN interférent conforme à l'invention, placée sous contrôle d'un promoteur approprié.

Les «Aptamères» sont une classe de molécule qui représente une alternative aux anticorps en terme de reconnaissance moléculaire. Les aptamères sont des séquences oligonucléotidiques ou oligopeptidiques qui possèdent la capacité de reconnaître pratiquement toutes les catégories de molécules cible avec une haute affinité et une forte spécificité. Ces ligands peuvent être isolés d'une banque de séquences aléatoires par mise en oeuvre de la méthode « SELEX » (pour « Systematic Evolution of Ligands by EXponential enrichment »), comme décrit dans Tuerk C. et L. Gold (Science, 1990, 249 (4968) : 505-10). La banque de séquence aléatoire peut être obtenue par synthèse chimique combinatoire de l'ADN. Dans cette bibliothèque, chaque membre est un oligomère linéaire, éventuellement modifié chimiquement, d'une séquence unique. Les modifications possibles, les utilisations et les avantages de cette classe de molécules ont été exposés par Jayasena SD, Clin. Chem., 1999, 45 (9) :1628-50. Les aptamères peptidiques sont des peptides conformationnels, en général d'une vingtaine de résidus, sélectionnés à partir de bibliothèques combinatoires par le système du double hybride (Colas et al., Nature, 1996, 380 : 548-50 ; Hoppe-Seyler et al., J. Steroid Biochem. Mol. Biol., 2001, 78(2) : 105-11)).

Le terme «ribozyme» désigne une molécule d'ARN possédant une activité enzymatique qui est capable de cliver d'autres molécules d'ARN distinctes, le clivage étant spécifique d'une séquence ribonucléotidique cible donnée. Dans le cas présent, la séquence ribonucléotidique cible est comprise dans la séquence de l'ARNm ou du pré-ARNm issu de la transcription du gène codant la protéine SAMHD1.

La composition vaccinale ou la composition immunogène selon l'invention comprendra au moins un antigène du rétrovirus contre lequel on souhaite diriger le vaccin, ou la séquence polynucléotidique codant cet antigène.

Lorsque la composition comprend une séquence polynucléotidique codant un antigène du rétrovirus, ladite séquence sera comprise dans tout vecteur d'expression permettant l'expression de l'antigène lorsque le vecteur est dans une cellule encaryote.

L'antigène d'un rétrovirus contre lequel on souhaite diriger le vaccin ou la réponse immunitaire sera de préférence sélectionné dans le groupe constitué par une protéine dudit rétrovirus ou un fragment de celle-ci, un vecteur d'expression comprenant la séquence polynucléotidique codant une protéine dudit rétrovirus ou un fragment de celle-ci, une pseudoparticule virale possédant les protéines structurales dudit rétrovirus, un antigène lipopeptidique comprenant des protéines ou des fragments peptidiques dudit rétrovirus, ledit rétrovirus sous forme inactivée ou sous forme atténuée, et un vecteur viral Lorsque l'antigène est une protéine virale, il s'agira de préférence d'une protéine structurale sélectionnée dans le groupe constitué des glycoprotéines d'enveloppe (gp120 et gp 41 dans le cas des VIH), de la protéine de capside, de la protéine de matrice, plus préférentiellement des glycoprotéines d'enveloppe.

Les vecteurs d'expressions comprenant la séquence polynucléotidique codant cet antigène sont notamment des vecteurs d'expression décrits dans l'article de revue de Kumar P. et al., Curr. Gene Ther., 11 (2) : 144-53, 2011. Ces vecteurs d'expression peuvent notamment être : un vecteur d'expression rétrovital, vecteur adénoviral, etc.

Le vecteur rétroviral sera un vecteur lentiviral de type VIH, VIS et VIF, dans la mesure où ces vecteurs ont la propriété de transduire les cellules qui ne se divisent pas telles que les monocytes et les cellules dendritiques, cibles principales du vaccin puisque se sont ces cellules que l'on souhaite activer précocement. Le vecteur lentiviral sera de préférence un vecteur VIH. Ce type de vecteur lentiviral est notamment décrit dans l'article de revue de Kumar P. et al., Curr. Gene Ther., 11 (2) : 144-53, 2011). De préférence, il s'agira d'un vecteur lentiviral de type pTrip tel que celui précédemment décrit par Royer-Leveau et al. (Journal of Virological Methods, 105 : 133-140, 2002).

On entend par « pseudoparticule virale » des particules virales (comprenant au moins les protéines structurales du rétrovirus) sans génome. Les techniques pour obtenir des pseudoparticules virales sont bien connues de l'homme du métier. On citera notamment la technique consistant à faire exprimer dans une cellule, notamment une cellule de mammifère, d'insecte, de levure, ou une bactérie, un vecteur d'expression codant les protéines structurales du rétrovirus.

On entend par « rétrovirus sous forme inactivé » toute particule virale qui est incapable d'infecter une cellule cible qui est normalement sensible et permissible à l'infection ou de réaliser un cycle de réplication dans la cellule cible qui est normalement sensible et permissible à l'infection.

Les rétrovirus sous forme inactivée peuvent être obtenus par les techniques bien connues de l'homme du métier. On citera notamment les rétrovirus inactivés par traitement à l'aldrithiol-2 ou inactivés par la chaleur (par exemple deux fois 30 minutes à 56 °C).

On entend par « rétrovirus sous forme atténuée » tout rétrovirus dont la capacité à compléter un cycle viral de réplication est inférieure à celle du même rétrovirus natif. Afin d'obtenir un rétrovirus atténué, il est possible de déléter certains gènes du rétrovirus, notamment des gènes accessoires : par exemple dans le cas du VIH il est possible de supprimer du génome viral les gènes *nef*, *vif*, *vpr* et/ou *vpu* pour atténuer le virus (voir Watkins DI. Top HIV Med;18(2): 35-6, 2010)

On entend par « antigène lipopeptidique comprenant des protéines ou des fragments peptidiques du rétrovirus » une protéine du rétrovirus ou un fragment de celle-ci qui a été modifiée à l'une des extrémités par addition d'un groupe lipidique. De préférence, le groupe lipidique est un groupe N-ε-palmitoyl-lysylamide. Des antigènes lipopeptidiques sont par exemple ceux décrits dans la publication Cobb et al. (Journal of Immunological Methods, 365 : 27-37, 2011).

On entend par « vecteur viral pseudotypé » une particule virale dont les glycoprotéines d'enveloppe proviennent du rétrovirus contre lequel on souhaite diriger le vaccin alors que le reste du virus, notamment les constituants internes, proviennent d'un autre virus.

Dans un mode de réalisation préféré, la composition vaccinale et la composition immunogène selon l'invention comprendront un rétrovirus sous forme atténuée ou un vecteur rétroviral, ainsi qu'au moins un inhibiteur de l'expression et/ou un de l'activité de la protéine SAMHD1.

Le rétrovirus contre lequel on souhaite diriger la composition vaccinale ou la composition immunogène peut être un lentivirus sélectionné dans le groupe constitué par:
- les lentivirus de primates (les virus de l'immunodéficience humaine, type 1 (VIH-1), les virus de l'immunodéficience humaine, type 2 (VIH-2), et les virus de l'immunodéficience simienne (VIS)).

Le lentivirus est plus préférentiellement un VIH, avantageusement un VIH-1.

L'antigène sera choisi afin qu'il soit de la même origine que le rétrovirus contre lequel on souhaite diriger la composition vaccinale ou la composition immunogène.

De préférence, l'antigène sera un antigène de lentivirus, avantageusement VIH-1.

La composition vaccinale et la composition immunogène peuvent être sous toute forme pouvant être administrée. Typiquement, les vaccins et les compositions immunogènes selon l'invention sont préparés sous des formes injectables (soit sous forme de solution liquide, soit sous forme de suspension), des formes solides convenables pour être mises en suspension ou en solution dans un liquide avant l'injection.

Des excipients pharmaceutiquement acceptables, c'est-à-dire n'ayant pas d'effet physiologiques néfastes pour le sujet, et compatibles avec la survie des mycobactéries présentent dans le vaccin, peuvent être utilisés. Parmi ces excipients, on citera l'eau, une solution saline (eau physiologique), le glycérol.

Le vaccin ou la composition immunogène peuvent également contenir des quantité mineures de substances auxiliaires, telles que des agents tamponnant le pH, ou des adjuvants qui ont pour fonction d'améliorer l'efficacité du vaccin en stimulant, activant, prolongeant, renforçant, potentialisant et/ou modulant la réponse immunitaire dirigée contre la mycobactérie présente dans le vaccin. Les adjuvants utilisés en médecine humaine, notamment ceux couramment utilisés dans le cas d'un vaccin vivant atténué, sont bien connu de l'homme du métier. On citera notamment l'hydroxyde ou le phosphate d'aluminium, le squalène, le tocophérol, l'hydroxyapathite, les adjuvants complets et incomplets de Freund, le bromure de diméthyldioctadécylammonium (DDA), les lymphokines (notamment l'IFN gamma, l'IL-2 et l'IL-12), la paroi issue de *M*. *bovis* souche BCG.

### MODULATEUR DE L'EXPRESSION ET/OU DE L'ACTIVITE DE SAMHD1

La présente invention à également pour objet un modulateur de l'expression et/ou de l'activité de SAMHD1 tel que défini dans les revendications pour son utilisation dans le traitement et/ou la prévention d'une maladie par un rétrovirus.

On entend par « modulateur de l'expression de la protéine SAMHD1 » tout composé capable soit d'augmenter soit d'inhiber l'expression de cette protéine. On entend par « expression de la protéine SAMHD1 » la transcription de l'ARNm à partir du gène codant SAMHD1 ou la traduction de l'ARNm en protéine SAMHD1 (SEQ ID NO : 1) à partir de l'ARNm. De même, le terme « modulateur de l'activité de la protéine SAMHD1 » signifie amplifier ou inhiber l'activité de la protéine SAMHD1.

Les modulateurs de l'expression et/ou de l'activité de SAMHD1 peuvent être isolés à partir des méthodes de criblages selon l'invention.

Lorsque le modulateur de l'expression et/ou de l'activité de SAMHD1 est un inhibiteur de l'expression et/ou l'activité de SAMHD1, il s'agit d'un inhibiteur tel que défini ci-dessus dans la composition vaccinale, c'est-à-dire un inhibiteur sélectionné dans le groupe constitué par une petite molécule chimique, un ARN interférent dirigé contre l'ARNm ou le pré-ARNm de SAMHD1, un oligonucléotide antisens ciblant le gène codant SAMHD1, un ribozyme dirigé contre l'ARNm ou le pré-ARNm de SAMHD1, un anticorps anti-SAMHD1 (de préférence un anticorps humanisé), ou un dérivé d'anticorps anti-SAMHD1, et un aptamère.

Lorsque l'inhibiteur agit au niveau de la protéine, et non au niveau du gène ou du transcrit de SAMHD1, il ciblera de préférence le domaine« HDc » de SAMHD1, et avantageusement le site actif du domaine phosphohydrolase de SAMHD1 afin d'inactiver cette activité.

Le modulateur de l'expression et/ou l'activité de SAMHD1 est utilisé dans une quantité thérapeutiquement efficace. Chez un sujet déjà infecté par un rétrovirus, on entend par « quantité thérapeutiquement efficace » une quantité de composé suffisante pour empêcher la maladie d'évoluer vers une aggravation, voire suffisante pour faire régresser la maladie. Chez un sujet non infecté, la « quantité thérapeutiquement efficace » est la quantité suffisante pour protéger un sujet qui serait mis en contact avec ledit rétrovirus et éviter la survenue de la maladie causée par ce rétrovirus. La quantité exacte de modulateur à administrer varie selon l'âge et le poids du sujet à traiter, le type de maladie, le stade de la maladie, l'état du sujet, le mode d'administration, la fréquence d'administration ainsi que les autres ingrédients présents dans la composition qui comprend l'inhibiteur.

La maladie est causée par un rétrovirus qui est un lentivirus sélectionné dans le groupe constitué par:
- les lentivirus de primates (les virus de l'immunodéficience humaine, type 1 (VIH-1), les virus de l'immunodéficience humaine, type 2 (VIH-2), et les virus de l'immunodéficience simienne (VIS)).

Plus préférentiellement, le rétrovirus causant la maladie est un lentivirus de primates choisi parmi les VIH-1, les VIH-2, et les VIS. Avantageusement, il s'agit d'un VIH-1.

### TRANSDUCTION RETROVIRALE ET KIT DE TRANSDUCTION

Les inventeurs ont montré que la protéine SAMHD1 est responsable de l'inhibition du cycle réplicatif de VIH-1 dans les cellules exprimant cette protéine, en particulier dans les cellules dendritiques et les cellules de la lignée monocytaire. Ainsi, l'inhibition de l'expression et/ou de l'activité de la protéine SAMHD1 permet d'augmenter l'efficacité de la transduction de ces cellules par des vecteurs rétroviraux.

Par conséquent, la présente invention a également pour objet l'utilisation *in vitro* ou *ex vivo* d'un inhibiteur de l'expression et/ou de l'activité de la protéine SAMHD1 pour augmenter la transduction des cellules exprimant SAMHD1 par un vecteur d'expression rétroviral, tel que défini dans les revendications, plus préférentiellement un vecteur d'expression VIH-1.

Un autre objet de la présente invention est relatif à un kit tel que défini dans les revendications pour transduire un vecteur d'expression rétroviral, de préférence un vecteur d'expression lentiviral, dans une cellule exprimant SAMHD1, de préférence une cellule de la lignée monocytaire ou une cellule dendritique, comprenant :
- un inhibiteur de l'expression et/ou de l'activité de la protéine SAMHD1 ;
- un vecteur d'expression lentiviral qui n'exprime pas naturellement la protéine Vpx, plus préférentiellement un vecteur d'expression VIH-1.

L'inhibiteur présent dans le kit peut être tout inhibiteur tel que décrit précédemment dans la présente demande.

Dans un mode de réalisation préféré de l'utilisation *in vitro* ou *ex vivo* et du kit selon l'invention, le vecteur d'expression rétroviral utilisé code une protéine d'intérêt que l'on souhaite exprimer dans la cellule exprimant SAMHD1.

Dans le cadre la présente invention, on entend par « transduction » ou « transduire » le transfert de l'ARN génomique d'un rétrovirus dans la cellule hôte lors de l'infection par un rétrovirus, suivie de la transcription inverse de l'ARN génomique en ADN double brin susceptible de s'intégrer dans le génome de la cellule hôte, et le cas échéant d'exprimer une protéine d'intérêt si le vecteur code une telle protéine.

Le vecteur d'expression rétroviral est un vecteur d'expression lentiviral sélectionné dans le groupe constitué par :
l es lentivirus de primates (les virus de l'immunodéficience humaine, type 1 (VIH-1), les virus de l'immunodéficience humaine, type 2 (VIH-2), et les virus de l'immunodéficience simienne (VIS)).

Le vecteur d'expression lentiviral est plus préférentiellement un virus de l'immunodéficience humaine de type 1 (VIH-1), un virus de l'immunodéficience humaine de type 2 (VIH-2) ou un virus de l'immunodéficience simienne (VIS). Avantageusement, il s'agit d'un VIH-1.

De préférence, les cellules exprimant SAMHD1 sont sélectionnées dans le groupe constitué des cellules dendritiques et des cellules de la lignée monocytaire, notamment les monocytes et les macrophages

La présente invention concerne également une méthode pour exprimer une protéine d'intérêt dans des cellules exprimant SAMHD1, telle que définie dans les revendications, cette méthode comprenant les étapes suivantes :
i) mettre en présence les cellules exprimant SAMHD1 avec un inhibiteur de l'expression et/ou de l'activité de la protéine SAMHD1 ;
ii) infecter les cellules exprimant SAMHD1 de l'étape i) avec un vecteur d'expression lentiviral codant une protéine d'intérêt.
iii) optionnellement, contrôler que les cellules exprimant SAMHD1 expriment bien la protéine d'intérêt, par exemple par Western blot ou immunofluorescence à l'aide d'anticorps spécifiques de la protéine d'intérêt.

Dans un mode de réalisation, les étapes i) et ii) sont conduites simultanément.

Dans un mode de réalisation particulier de la méthode selon l'invention, les cellules exprimant SAMHD1 ont été obtenues à partir d'un sujet, par exemple à partir du sang périphérique de ce sujet. Elles peuvent par exemple être isolées par leukapherèse (voir Cobb et al., Journal of Immunological Methods, 365 : 27-37, 2011), ou triées par cytométrie en flux, notamment sur la base de leur granulométrie et/ou de marqueurs de surface spécifiques de ces cellules. Lorsqu'il s'agit de cellules dendritiques, les marqueurs spécifiques sont notamment CD86, CD80, CD83. Dans le cas, des monocytes et des macrophages, on citera notamment le marqueur CD14.

Dans un mode de réalisation avantageux, la méthode comprend une dernière étape dans laquelle les cellules exprimant SAMHD1 provenant d'un sujet obtenues aux étapes ii) (ou iii)) sont réimplantées chez ledit sujet. Cette méthode est particulièrement intéressante dans le cadre d'une thérapie génique visant à faire exprimer une protéine d'intérêt chez le sujet par les cellules du sujet exprimant SAMHD1, de préférence par les cellules dendritiques et/ou cellules de la lignée monocytaire.

Un autre objet de la présente invention est relatif à l'utilisation telle que définie dans les revendications de cellules exprimant SAMHD1 codant une protéine d'intérêt susceptibles d'être obtenues par la méthode pour exprimer une protéine d'intérêt selon l'invention pour son utilisation comme médicament pour traiter et/ou prévenir une maladie pour laquelle l'expression de la protéine d'intérêt permet prévention, la guérison, la diminution des symptômes de ladite maladie et/ou l'amélioration de la santé d'un sujet souffrant de cette maladie.

La protéine d'intérêt pourra notamment être un antigène de microorganisme, par exemple un antigène bactérien, viral ou de levure, ce qui permettra de traiter ou prévenir une maladie dans laquelle le microorganisme est impliqué. La protéine pourra également être un antigène spécifiquement exprimé par des cellules cancéreuses, les cellules serons alors utilisées dans le cadre d'une thérapie antitumorale.

### INDUCTION DE L'EXPRESSION D'INTERFERON DE TYPE 1

Les inventeurs ont maintenant montré que l'inhibition de l'expression de SAMHD1 par interférence ARN dans les cellules dendritiques, en l'absence de toute infection, activait l'expression et la sécrétion d'interférons de type 1.

On entend par « interférons de type 1 » les interférons alpha et beta.

Ainsi, la présente spécification décrit l'utilisation *in vitro* ou *ex vivo* d'un inhibiteur de l'expression et/ou de l'activité de la protéine SAMHD1 (SEQ ID NO : 1) pour induire une expression d'interférons de type 1 dans des cellules exprimant naturellement la protéine SAMHD1.

En outre, puisque les interférons de type 1 sont impliqués dans la réponse immunitaire antivirale innée, est également décrit un composé choisi parmi i) un inhibiteur de l'expression de la protéine SAMHD1, et ii) un inhibiteur de l'activité de la protéine SAMHD1 pour son utilisation comme médicament pour induire une expression d'interférons de type 1 dans des cellules exprimant naturellement la protéine SAMHD1, de préférence pour induire une réponse immunitaire innée lors d'une infection virale, notamment une infection par un rétrovirus, par exemple une infection par VIH.

Les cellules exprimant naturellement la protéine SAMHD1 sont de préférence des cellules dendritiques et/ou des cellules de la lignée monocytaire.

### INDUCTION D'UNE RESISTANTE A L'INFECTION PAR UN RETROVIRUS

Un autre objet de l'invention concerne une méthode *in vivo*, *in vitro* ou *ex vivo* telle que définie dans les revendications pour induire une résistance à l'infection par un lentivirus, chez une cellule n'exprimant pas naturellement la protéine SAMHD1, ladite méthode comprenant les étapes suivantes :
- introduire une séquence codant la protéine SAMHD1 dans ladite cellule n'exprimant pas naturellement la protéine SAMHD1 ;
- optionnellement vérifier l'expression de SAMHD1 ;
les cellules exprimant la protéine SAMHD1 étant résistante à l'infection par un rétrovirus.

La séquence codant la protéine SAMHD1 peut être le gène ou l'ADNc codant SAMHD1.

Dans le cadre de la présente invention, on entend par « une résistance à l'infection par un rétrovirus » une diminution de la réplication du rétrovirus dans la cellule en comparaison du taux de réplication observé dans une même cellule n'exprimant pas SAMHD1 (c'est-à-dire une cellule dans laquelle le gène codant SAMHD1 n'a pas été introduit). De préférence, la diminution de la réplication du rétrovirus sera d'au moins 10%, préférentiellement de 30%, plus préférentiellement d'au moins 50%, et avantageusement d'au moins 70%, 75%, 80%, 85%, 90%, 95%, ou 100%.

La réplication du rétrovirus pourra être mesurée par les techniques bien connues de l'homme du métier. Par exemple, la mesure de la réplication d'un rétrovirus pourra être réalisée par PCR quantitative en utilisant des amorces s'hybridant spécifiquement à la séquence dudit rétrovirus, par quantification de l'activité transcriptase inverse et/ou par quantification des antigènes viraux.

La présente invention concerne également une méthode telle que définie dans les revendications pour traiter ou prévenir une infection par un rétrovirus chez un sujet, cette méthode comprenant les étapes suivantes :
i) introduire une séquence codant la protéine SAMHD1 dans une cellule du sujet à traiter, ladite cellule n'exprimant pas naturellement la protéine SAMHD1 et étant une cellule cible du rétrovirus lors de l'infection ou une cellule qui est capable de se différencier en une cellule cible du rétrovirus ;
ii) optionnellement contrôler l'expression de SAMHD1 dans les cellules dans lesquelles la séquence du gène codant SAMHD1 a été introduite, les cellules exprimant la protéine SAMHD1 étant résistantes à l'infection par un rétrovirus ; et
iii) réimplanter la cellule exprimant SAMHD1 obtenue à l'étape i) (ou ii)) chez le sujet.

On entend par « cellule cible du rétrovirus lors de l'infection » toute cellule qui est naturellement infectée par le virus. Par exemple, dans le cas d'une infection par VIH, on citera notamment les lymphocytes T CD4+.

On entend par « réimplanter » l'administration de la cellule au sujet. Dans le cas de cellules souches CD34+, il s'agit de la reconstitution par des cellules CD34+ réinjectées dans la moelle osseuse du patient préparé à recevoir ces cellules (voir l'article de revue de Serrano et al., Curr HIV/AIDS Rep., 7(3): 175-84, 2010).

Un autre objet de la présente invention est relatif à une cellule n'exprimant pas naturellement la protéine SAMHD1 dans laquelle la séquence codant la protéine SAMHD1 a été introduite, ladite cellule étant une cellule cible du rétrovirus lors de l'infection ou une cellule qui est capable de se différencier en une cellule cible du rétrovirus, pour son utilisation comme médicament pour traiter ou prévenir chez un sujet une maladie causée par un rétrovirus.

Dans un mode de réalisation particulièrement avantageux, la cellule est une cellule autologue, c'est-à-dire qu'elle provient du sujet chez qui la cellule sera utilisée comme médicament pour traiter ou prévenir une maladie causée par un rétrovirus. Ainsi, la présente invention porte également sur un cellule d'un sujet n'exprimant pas naturellement la protéine SAMHD1 dans laquelle la séquence codant la protéine SAMHD1 a été introduite, ladite cellule étant une cellule cible du rétrovirus lors de l'infection ou une cellule qui est capable de se différencier en une cellule cible du rétrovirus, pour son utilisation comme médicament pour traiter ou prévenir une maladie causée par un rétrovirus chez ce sujet.

Le sujet sera de préférence un humain ou un mammifère non humain, notamment un primate non humain, un chat, une chèvre.

En règle générale, le sujet sera choisi en fonction du rétrovirus et de la spécificité d'hôte de ce rétrovirus. Par exemple, pour le VIH, le sujet sera un humain, pour le VIF le sujet sera un chat, etc.

Dans le cadre de la méthode *in vivo, in vitro* ou *ex vivo* pour induire une résistance à l'infection par un rétrovirus, de la méthode pour traiter ou prévenir une infection par un rétrovirus chez un sujet, et des utilisations d'une cellule n'exprimant pas naturellement la protéine SAMHD1 dans laquelle la séquence codant la protéine SAMHD1 a été introduite ci-dessus, le rétrovirus sera notamment un lentivirus sélectionné dans le groupe constitué par :
l es lentivirus de primates (les virus de l'immunodéficience humaine, type 1 (VIH-1), les virus de l'immunodéficience humaine, type 2 (VIH-2), et les virus de l'immunodéficience simienne (VIS)).

Le lentivirus est plus préférentiellement un virus de l'immunodéficience humaine de type 1 (VIH-1), un virus de l'immunodéficience humaine de type 2 (VIH-2) ou un virus de l'immunodéficience simienne (VIS). Avantageusement, il s'agit d'un VIH-1 ou d'un VIS qui n'exprime pas naturellement la protéine Vpx.

La cellule n'exprimant pas naturellement la protéine SAMHD1 est une cellule souche hématopoïétique capable de se différencier i) en cellules souches lymphoïdes qui à son tour se divisera pour donner les cellules lymphoïdes, notamment les lymphocytes T CD4+, les lymphocytes T CD8+, les lymphocytes B, les cellules dendritiques plasmoïdes, et ii) en cellules souches myéloïdes qui à son tour se divisera pour donner les cellules de la lignée myéloïde, notamment les monocytes.
Les cellules souches hématopoïétiques sont caractérisées notamment par l'expression des marqueurs immunologiques CD34 et Thy1 (cellules CD34+ et Thy1+) et l'absence d'expression du marqueur CD33 (cellules CD33-). Ces cellules peuvent être isolées par les techniques bien connues par l'homme du métier, notamment à partir de cellule de moelle osseuse, mais également de sang périphérique, en triant les cellules par cytométrie en flux sur la base des marqueurs de surface, notamment CD34.

Dans un mode de réalisation préféré, l'introduction de la séquence codant la protéine SAMHD1 dans ladite cellule n'exprimant pas naturellement la protéine SAMHD1 est réalisée à l'aide d'un vecteur de transfert de gène couramment utilisé en thérapie génique. On citera notamment les vecteurs viraux tels que les vecteurs adénoviraux et rétroviraux, ainsi que les vecteurs non viraux tels que des vecteurs d'expression plasmidique (Elsabahy et al., Curr. Drug. Deliv., 2011 Feb 3., PMID:21291381).

Lorsque l'on souhaite que le gène codant la protéine SAMHD1 soit intégré dans le génome de la cellule hôte, on utilisera un vecteur rétroviral, de préférence un vecteur d'expression lentiviral.

Un autre objet de l'invention concerne des cellules exprimant ou n'exprimant pas (ou faiblement) naturellement la protéine SAMHD1 dans laquelle la séquence codant la protéine SAMHD1 a été introduite et qui exprime donc une protéine SAMHD1 recombinante. Les cellules sont des cellules qui n'exprimaient pas naturellement la protéine SAMHD1 ou qui n'expriment naturellement que faiblement cette protéine : on citera notamment les lymphocytes T CD4+ et les cellules souches.

L'introduction de la séquence codant la protéine SAMHD1 dans lesdites cellules exprimant ou n'exprimant pas (ou faiblement) naturellement la protéine SAMHD1 est réalisée à l'aide de vecteurs d'expression bien connus de l'homme du métier, par exemple un vecteur rétroviral comprenant cette séquence.

La cellule n'exprimant pas (ou faiblement) naturellement la protéine SAMHD1 est une cellule souche hématopoïétique capable de se différencier i) en cellules souches lymphoïdes qui à son tour se divisera pour donner les cellules lymphoïdes, notamment les lymphocytes T CD4+, les lymphocytes T CD8+, les lymphocytes B, les cellules dendritiques plasmoïdes, et ii) en cellules souches myéloïdes qui à son tour se divisera pour donner les cellules de la lignée myéloïde, notamment les monocytes.

La présente invention sera mieux comprise à l'aide du complément de description qui va suivre, qui se réfère à des exemples non-limitatifs montrant que l'inhibition de l'expression de SAMHD1 chez les cellules dendritiques induit l'expression des interférons de type 1 et rend d'autre part ces cellules plus sensibles à l'infection par VIH-1. Description des Figures :

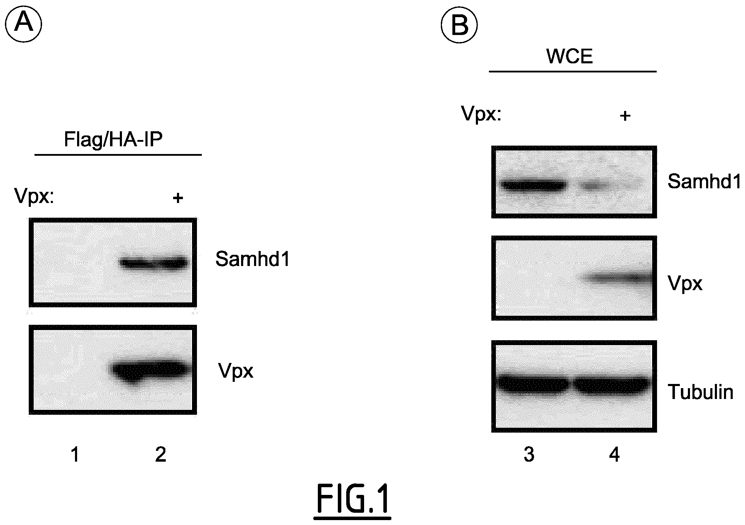

Figure 1 : La Figure 1.A présente une immunoprécipitation réalisée sur des lysats de cellules THP-1 exprimant (ligne 2) ou pas (ligne 1) Vpx fusionnée avec les peptides Flag et HA utilisant successivement des anticorps anti-Flag puis anti-HA, et couplées à des élutions en conditions natives par compétition avec des excès de peptide Flag et HA. Les protéines ainsi éluées sont analysées par western blot à l'aide d'un anticorps anti-SAMHD1. La Figure 1.B présente l'analyse par western blot à l'aide d'anticorps anti-SAMHD1, Flag ou Tubulin d'extraits totaux (« WCE ») préparés à partir des cellules exprimant (ligne 4) ou n'exprimant pas (ligne 3) eVpx.

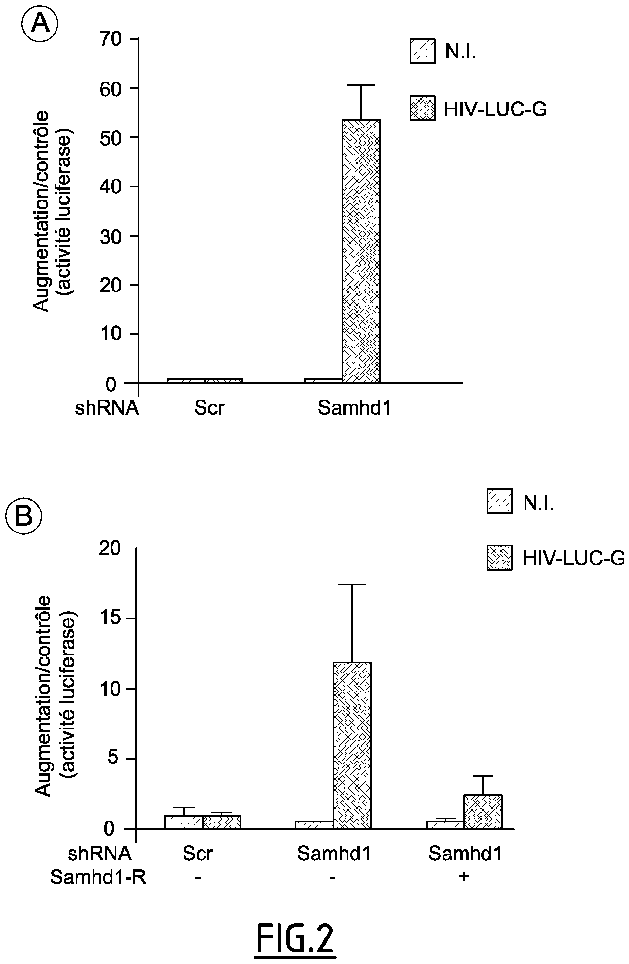

Figure 2 : La Figures 2.A est un histogramme montrant l'infection par un VIH-1 exprimant la luciférase (HIV-LUC-G) (infection mesurée par quantification de l'activité luciférase) de cellules THP1 exprimant naturellement SAMHD1 qui ont été transduites par un shRNA contrôle non spécifique de SAMHD1 (Scr) ou un shRNA spécifique de SAMHD1 (brin sens 5'CGCAGGAUGGCGAUGUUAU3' ; brin anti-sens 5'AUAACAUCGCCAUCCUGCG3') pour inhiber son expression. La Figures 2.B est un histogramme montrant l'infection par HIV-LUC-G de cellules THP1 exprimant naturellement SAMHD1 qui ont été transduites i) par un shRNA contrôle non spécifique de SAMHD1 (Scr), ii) un shRNA spécifique de SAMHD1 (Samhd1), ou iii) un shRNA spécifique de SAMHD1 (Samhd1) et vecteur codant une forme de SAMHD1 résistante au RNAi (Samhd1-R).
   Les colonnes « N.I. » représentent les cellules non infectées.

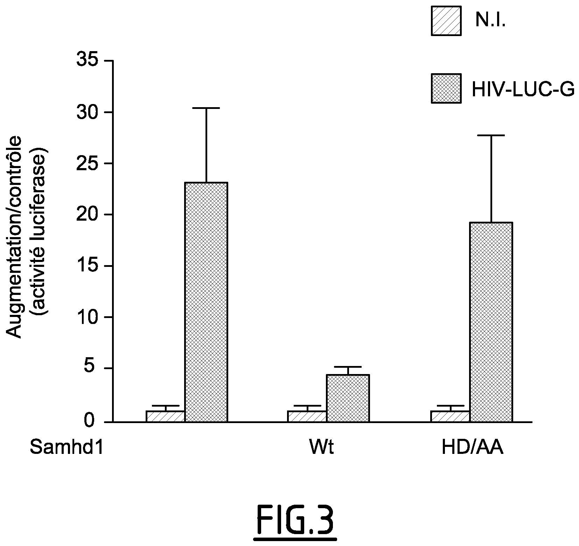

Figure 3 : La Figure 3 est un histogramme montrant l'implication du domaine « HDc » de la protéine SAMHD1 dans l'inhibition de l'infection par VIH. Les colonnes « WT » montre les résultats obtenus lorsque des cellules HeLa (permissives au VIH-1) surexpriment SAMHD1. Les colonnes « HD/AA » montrent les résultats obtenus lorsque des cellules HeLa surexpriment une forme mutée de SAMHD1 ne possédant pas d'activité phosphohydrolase. Les colonnes « HIV-LUC-G » représentent les résultats obtenus lors d'une infection des cellules par un VIH-1 exprimant la luciférase. Les colonnes « N.I. » représentent les cellules non infectées.

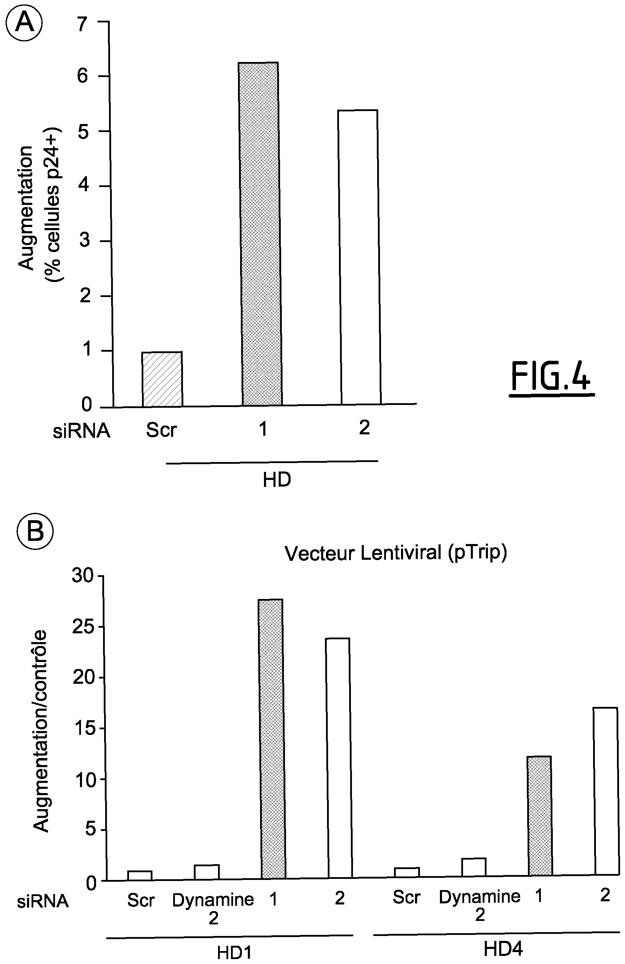

Figure 4 : La Figures 4.A est un histogramme montrant l'infection de cellules dendritiques primaires provenant d'un donneur sain (« HD ») par un VIH-1 lorsque ces cellules sont traitées ou non par des siRNA ciblant spécifiquement SAMHD1 (mesure réalisée par quantification du nombre de cellules exprimant la protéine virale p24). La colonne « Scr » représente le résultat obtenu lorsque les cellules sont traitées par un siRNA contrôle non spécifique de SAMHD1, alors que les colonnes « 1 » et « 2 » représentent le résultat obtenu lorsque les cellules sont traitées par deux siRNA différents ciblant spécifiquement SAMHD1 (siRNA correspondant respectivement aux SEQ ID Nos : 3 et 4). La Figures 4.B est un histogramme montrant l'infection de cellules dendritiques primaires provenant de donneurs sains « HD1 » ou « HD2 » par un vecteur lentiviral pTrip lorsque ces cellules sont traitées ou non par des siRNA ciblant spécifiquement SAMHD1 La colonne « Scr » représente le résultat obtenu lorsque les cellules sont traitées par un siRNA contrôle non spécifique de SAMHD1, la colonne « Dynamine 2 » représente le résultat obtenu lorsque les cellules sont traitées par un siRNA spécifique de la dynamine 2, alors que les colonnes « 1 » et « 2 » représentent le résultat obtenu lorsque les cellules sont traitées par deux siRNA différents ciblant spécifiquement SAMHD1.

### Exemple 1 : Matériels et Méthodes

Les lignées stables THP-1 exprimant de manière stable Vpx étiqueté avec les peptides Flag et HA (ci-après abrégé par eVpx) ont été générées à l'aide d'un vecteur rétroviral MMLV qui contient une unité de transcription bicistronique permettant l'expression de la protéine d'intérêt et d'un marqueur de sélection (la chaîne α du récepteur à l'IL2-IL2Rα).

Les cellules transduites avec ce vecteur rétroviral ont été sélectionnées avec des billes magnétiques couplées à un anticorps reconnaissant l'IL2Rα.

Les extraits cellulaires permettant de voir l'effet de Vpx sur la dégradation de Samhd1 ont été réalisés avec un tampon de lyse contenant 0,5% de triton, 150 mM de NaCl, 10 mM de KCL, 1,5 mM de MgCl2, 0,5 mM d'EDTA, 10 mM de β-mercaptoethanol et 0,5 mM de PMSF.

Les immunoprécipitations de eVpx ont été réalisées grâce à un anticorps reconnaissant spécifiquement l'étiquette Flag ou HA. Les immunoprécipitats sont élués en conditions natives grâce à un excès de peptide Flag ou HA.

Les infections ont été réalisées soit i) avec un VIH comportant le gène codant la Luciférase à la place de celui codant pour Nef pseudotypé avec la glycoprotéine d'enveloppe du virus de la stomatite vésiculaire (HIV-LUC-G), soit ii) avec un VIH sauvage pseudotypé avec la glycoprotéine d'enveloppe du virus de la stomatite vésiculaire (HIV-G).

Les doses de virus utilisées pour réaliser les infections ont été déterminées par dosage de la protéine p24 (composante de Gag) contenue dans les cellules productrices de virus et normalisées avant les infections.

Pour les expériences avec le HIV-LUC-G, des doses de 1 ng (pour les cellules HeLa) ou de 100 ng (pour les cellules THP-1) ont été utilisées. Pour les expériences avec le HIV-G une dose de 100 ng a été utilisée.

Les expériences d'ARN interférence ont été réalisées en utilisant les siRNA correspondant aux SEQ ID Nos : 3 (5'GAUUCAUUGUGGCCAUAUA3') et 4 (5'CAACCAGAGCUGCAGAUAA3') (dans les cellules dendritiques primaires), ou un shRNA (brin sens 5'CGCAGGAUGGCGAUGUUAU3' (SEQ ID NO : 5) ; brin anti-sens 5'AUAACAUCGCCAUCCUGCG3' (SEQ ID NO: 6) (dans les cellules THP-1), ciblant spécifiquement SAMHD1. Des lignées stables de cellules THP-1 ont été établies grâce à un marqueur de sélection à la puromycine compris dans le vecteur codant pour le shRNA.

Pour les expériences réalisées sur les cellules dendritiques primaires provenant du donneur sain (HD) l'extinction de l'expression de Samhd1 a été réalisée en transitoire.

Les mutants de Samhd1 ont été générés en accord avec le protocole standard de mutagénèse dirigée. Ces mutants ainsi que la séquence codant Samhd1 sauvage ont été introduites dans un vecteur rétroviral.

L'expression de Samhd1 sauvage ainsi que des mutants qui en dérivent a été réalisée grâce à une transduction en utilisant ces vecteurs rétroviraux.

Les cellules dendritiques primaires ont été purifiées à partir de cellules mononuclées du sang périphérique de donneurs sains par tri CD14 suivi d'une stimulation avec des cytokines (GM-CSF et IL-4) pendant 6 jours avant leur utilisation expérimentale.

### Exemple 2 : Résultats

La littérature montre l'existence d'un facteur cellulaire non identifié, exprimé dans les cellules dendritique et les macrophages, capable de restreindre l'infection par les lentivirus et en particulier le VIH-1. L'action de ce facteur est contrecarrée par la protéine virale Vpx exprimée par le VIH-2 et par certaine souche du SIV. La protéine Vpx induirait la dégradation de ce facteur en le ciblant au protéasome.

Afin de mettre en évidence ce facteur cellulaire, des cellules THP1 non permissives exprimant Vpx fusionné aux épitopes Flag et HA (eVpx) ont été générées.

Les cellules THP1 ainsi générées ont été lysées puis une technique de double immunopurification a alors été appliquée sur le lysat cellulaire à l'aide d'anticorps dirigés contre l'épitope Flag suivie d'une deuxième immunopurification à l'aide d'anticorps spécifique de l'épitope HA.

Les produits d'immunoprécipitation ont été analysés sur gel de polyacrylamide suivie d'une coloration des protéines au bleu de coomassie. Les protéines interagissant avec eVpx ont été identifiées par spéctrométrie de masse.

La protéine SAMHD1 a alors été identifiée comme interactant majeur de Vpx.

Cette interaction a été validée par immunoprécipitation de la protéine Vpx suivie d'une détection de la protéine SAMHD1 à l'aide d'anticorps spécifiques. Pour cela, des cellules non-permissives THP1 ont été transduites par un vecteur d'expression de Vpx étiqueté Flag et HA (eVpx) (cf. Figure 1, ligne 2) ou un vecteur vide (cf. Figure 1, ligne 1).

Les extraits cellulaires ont été préparés et soumis à une double immunoprécipitation à l'aide d'anticorps spécifique de l'étiquette Flag et HA. Les immunoprécipitats ont été analysés sur gel d'acrylamide et la présence de SAMHD1 a été analysée par western blot en utilisant un anticorps spécifique de SAMHD1 (cf. Figure 1.A). Les extraits totaux (« WCE ») préparés à partir des cellules exprimant (cf. Figure 1, ligne 4) ou n'exprimant pas (cf. Figure 1, ligne 3) eVpx ont été analysés sur gel d'acrylamide suivie d'un western blot à l'aide d'anticorps anti-SAMHD1, Flag ou Tubulin (cf. Figure 1.B) comme indiqué.

Les résultats d'immunoprécipitation sont présentés à la Figure 1.

La Figure 1 montre que Vpx interagit avec la protéine SAMHD1 endogène (cf. Figure 1.A). La quantification de SAMHD1 dans des extraits de cellules exprimant ou n'exprimant pas Vpx démontre également que Vpx induit la dégradation de SAMHD1 (Figure 1.B).

Des expériences ont ensuite été menées afin de savoir si l'inhibition de l'expression de SAMHD1 à l'aide d'un shRNA spécifique rend les cellules THP1 permissives à l'infection par le VIH-1.

Pour ce faire, des cellules THP1 ont été transduites par un shRNA contrôle (Scr) (témoin négatif) ou un shRNA spécifique de SAMHD1 pour inhiber son expression. Les THP1 transduites ont été infectées par du VIH-1 exprimant la luciférase (colonne HIV-LUC-G) ou non infectées (colonne « N.I. »). L'infection a été quantifiée par dosage de l'activité luciférase dans les extraits cellulaires.

Les résultats sont présentés à la Figure 2.

L'inhibition de l'expression de SAMHD1 par interférence à l'ARN (ARNi) dans les cellules non permissives au VIH-1 (cellule de la lignée monocytaire humaine THP1 différenciée) les rend permissives (Figure 2.A). La surexpression d'une forme de SAMHD1 résistante au RNAi (protéine Samhd1-R) dans les cellules où le niveau de SAMHD1 a été réduit par RNAi permet à ces cellules de retrouver leur phénotype de non permissivité au VIH (Figure 2.B).

L'effet de la surexpression de SAMHD1 sur l'infection de cellules qui n'expriment pas constitutivement cette protéine, à savoir des cellules HeLa, a ensuite été évalué.

Pour cela, les cellules HeLa ont été transfectées par un vecteur vide ou un vecteur exprimant la protéine SAMHD1 ou un SAMHD1 muté dans son domaine « HDc » portant l'activité l'activité phosphohydrolase (RNase) (Samhd1-HD/AA). Les cellules transfectées ont été infectées par le VIH-1 exprimant la luciférase. L'infection des cellules a été mesurée par dosage de l'activité luciférase.

Les résultats sont présentés à la Figure 3.

Cette figure montre que la surexpression de SAMHD1 dans des cellules permissives au VIH-1 (cellules HeLa) les rend non-permissives (Figure 3, colonne « WT »). De façon intéressante, la surexpression d'une forme mutée de SAMHD1 ne possédant pas d'activité phosphohydrolase n'a aucun effet sur la réplication virale (Figure 3, colonnes « HD/AA »). Ce résultat montre l'importance de l'activité enzymatique de SAMHD1 (l'activité phosphohydrolase) dans la restriction du VIH-1.

L'implication de SAMHD1 dans des cellules dendritiques primaires dérivées de cellules monocytaires isolées à partir du sang de donneur sain a été testée.

Pour cela, des cellules périphériques du sang d'un donneur sain (HD) ont été purifiées et différenciées en cellules dendritiques *ex vivo.* Ces cellules ont été traitées par deux siRNA différents ciblant spécifiquement SAMHD1 (siRNA correspondant respectivement aux SEQ ID Nos : 3 et 4) (cf. Figure 4, colonnes 1 et 2, respectivement), ou non traitée (ligne Scr), puis le pourcentage de cellules p24 positives a été déterminé par cytométrie en flux.

Ces résultats sont présentés à la Figure 4.A.

Cette figure montre que le traitement par l'un ou l'autre des siRNA ciblant spécifiquement SAMHD1 résulte en une augmentation de la production de Gag (p24) dans les cellules suite à l'infection avec un virus sauvage, ce qui traduit une augmentation de la transduction virale et de l'infectivité des cellules primaires par VIH-1.

Des expériences similaires ont été menées à l'aide de cellules dendritiques primaires dérivées de cellules monocytaires isolées à partir du sang de deux donneurs sains (« HD1 » et « HD4 ») en utilisant un vecteur lentiviral pTrip précédemment décrit par Royer-Leveau et al. (Journal of Virological Methods, 105 : 133-140, 2002).

Ces résultats sont présentés à la Figure 4.B.

Cette figure montre clairement que l'infection par le vecteur pTrip est fortement augmentée dans les cellules dendritiques primaires des deux donneurs sains, et ce qu'elle que soit le siRNA ciblant spécifiquement SAMHD1 utilisé. Ce résultat indique que la transduction du vecteur lentiviral pTrip est augmentée dans les cellules dendritiques lorsque l'expression de SAMHD1 est inhibée.

En conclusion, la protéine SAMHD1 a été identifiée comme le facteur de restriction aux infections par les lentivirus exprimé par les cellules dendritiques et les macrophages dont l'activité est contrecarrée par Vpx. Les cellules dendritiques et les macrophages jouent un rôle crucial dans la mise en place de la réponse immunitaire. Ce sont des cellules présentatrices de l'antigène. Elles sont nécessaires pour une réponse coordonnée et durable permettant la protection de l'organisme contre l'agent infectieux. Plusieurs études suggèrent que l'inefficacité des vaccins actuels est principalement due à la restriction de l'infection par VIH-1 des cellules dendritiques et des macrophages. L'identification de SAMHD1 comme le facteur de restriction exprimé par ces cellules est donc crucial pour l'élaboration d'un vaccin efficace contre les lentivirus en général et le VIH-1 en particulier. De plus, cette découverte permet d'améliorer la transduction des cellules exprimant la protéine SAMHD1 par les vecteurs lentiviraux à des fins de thérapie génique ou pour des expériences *in vitro.*

## Revendications

1. Méthode *in vitro* de criblage de composés candidats susceptibles d'être utilisés pour le traitement préventif et/ou curatif d'une maladie causée par un rétrovirus, comprenant les étapes suivantes :
- mettre en contact un composé candidat avec une cellule exprimant SAMHD1, ou avec la protéine SAMHD1 ; et
- déterminer si le composé candidat module l'expression et/ou l'activité de la protéine SAMHD1 ;
dans laquelle ledit composé candidat qui module l'expression et/ou l'activité de la protéine SAMHD1 est identifié comme composé candidat susceptible d'être utilisé pour le traitement préventif et/ou curatif d'une maladie causée par un rétrovirus,
ledit rétrovirus appartenant au genre lentivirus et étant sélectionné dans le groupe constitué des espèces constituées des virus de l'immunodéficience humaine de type 1 (VIH-1), des virus de l'immunodéficience humaine de type 2 (VIH-2) et des virus de l'immunodéficience simienne (VIS).

2. Méthode *in vitro* de criblage selon la revendication 1, **caractérisée en ce que** :
- la détermination de la capacité du composé candidat à moduler l'expression de SAMHD1 est réalisée en quantifiant l'ARNm SAMHD1 et/ou la protéine SAMHD1 ; et
- la détermination de la capacité du composé candidat à moduler l'activité de SAMHD1 est réalisée en mesurant l'activité phosphohydrolase de SAMHD1.

3. Inhibiteur de l'expression et/ou de l'activité de SAMHD1 pour son utilisation dans le traitement et/ou la prévention d'une maladie causée par un rétrovirus, ledit rétrovirus étant un lentivirus sélectionné dans le groupe constitué des espèces constituées des virus de l'immunodéficience humaine de type 1 (VIH-1), des virus de l'immunodéficience humaine de type 2 (VIH-2) et des virus de l'immunodéficience simienne (VIS), et ledit inhibiteur étant sélectionné dans le groupe constitué par un ARN interférent dirigé contre l'ARNm ou le pré-ARNm de SAMHD1, un oligonucléotide antisens ciblant le gène codant SAMHD1, un ribozyme dirigé contre l'ARNm ou le pré-ARNm de SAMHD1, un anticorps anti-SAMHD1, un dérivé d'anticorps anti-SAMHD1 et un aptamère.

4. Composition immunogène ou vaccinale comprenant :
i) un inhibiteur de l'expression et/ou de l'activité de la protéine SAMHD1 sélectionné dans le groupe constitué par des anticorps anti-SAMHD1, des ARN interférents dirigés contre l'ARNm ou le pré-ARNm de SAMHD1, des oligonucléotides antisens ciblant le gène codant SAMHD1, des ribozymes dirigés contre l'ARNm ou le pré-ARNm de SAMHD1 et des aptamères ; et
ii) au moins un antigène d'un rétrovirus ou une séquence polynucléotidique codant cet antigène, le rétrovirus étant un lentivirus sélectionné dans le groupe constitué des virus de l'immunodéficience humaine de type 1 (VIH-1), des virus de l'immunodéficience humaine de type 2 (VIH-2) et des virus de l'immunodéficience simienne (VIS), et l'antigène étant sélectionné dans le groupe constitué des glycoprotéines d'enveloppe, de la protéine de capside, et de la protéine de matrice.

5. Composition immunogène ou vaccinale selon la revendication 4, **caractérisée en ce que** l'antigène est sélectionné dans le groupe constitué par : une protéine dudit rétrovirus ou un fragment de celle-ci, un vecteur d'expression comprenant la séquence polynucléotidique codant une protéine dudit rétrovirus ou un fragment de celle-ci, une pseudoparticule virale possédant les protéines structurales dudit rétrovirus, un antigène lipopeptidique comprenant des protéines ou des fragments peptidiques dudit rétrovirus, ledit rétrovirus sous forme inactivée ou sous forme atténuée, et un vecteur viral pseudotypé.

6. Utilisation *in vitro* ou *ex vivo* d'un inhibiteur de l'expression et/ou de l'activité de la protéine SAMHD1 sélectionné dans le groupe constitué par des anticorps anti-SAMHD1, des ARN interférents dirigés contre l'ARNm ou le pré-ARNm de SAMHD1, des oligonucléotides antisens ciblant le gène codant SAMHD1, des ribozymes dirigés contre l'ARNm ou le pré-ARNm de SAMHD1 et des aptamères pour augmenter la transduction de cellules exprimant SAMHD1 par un vecteur rétroviral, le vecteur rétroviral étant un virus de l'immunodéficience humaine de type 1 (VIH-1), un virus de l'immunodéficience humaine de type 2 (VIH-2) ou un virus de l'immunodéficience simienne (VIS).

7. Kit pour transduire un vecteur rétroviral dans une cellule exprimant naturellement SAMHD1, comprenant :
- un inhibiteur de l'expression et/ou de l'activité de la protéine SAMHD1 sélectionné dans le groupe constitué par des anticorps anti-SAMHD1, des ARN interférents dirigés contre l'ARNm ou le pré-ARNm de SAMHD1, des oligonucléotides antisens ciblant le gène codant SAMHD1, des ribozymes dirigés contre l'ARNm ou le pré-ARNm de SAMHD1 et des aptamères ;
- un vecteur rétroviral qui n'exprime pas naturellement la protéine Vpx,
le vecteur rétroviral étant un vecteur lentiviral sélectionné dans le groupe constitué par un virus de l'immunodéficience humaine de type 1 (VIH-1), un virus de l'immunodéficience humaine de type 2 (VIH-2) et un virus de l'immunodéficience simienne (VIS).

8. Méthode *in vitro* ou *ex vivo* pour induire une résistance à l'infection par un rétrovirus dans une cellule n'exprimant pas naturellement la protéine SAMHD1, ladite méthode comprenant une étape consistant à introduire une séquence codant la protéine SAMHD1 dans ladite cellule n'exprimant pas naturellement la protéine SAMHD1, les cellules exprimant la protéine SAMHD1 étant résistantes à l'infection par un rétrovirus, ledit rétrovirus étant un lentivirus sélectionné dans le groupe constitué par un virus de l'immunodéficience humaine de type 1 (VIH-1), un virus de l'immunodéficience humaine de type 2 (VIH-2) et un virus de l'immunodéficience simienne (VIS).

9. Une cellule n'exprimant pas naturellement la protéine SAMHD1 dans laquelle la séquence codant la protéine SAMHD1 a été introduite, ladite cellule étant une cellule cible d'un rétrovirus lors d'une infection ou une cellule qui est capable de se différencier en une cellule cible du rétrovirus, pour son utilisation comme médicament pour traiter ou prévenir une maladie causée par un rétrovirus, ledit rétrovirus étant un lentivirus sélectionné dans le groupe constitué par un virus de l'immunodéficience humaine de type 1 (VIH-1), un virus de l'immunodéficience humaine de type 2 (VIH-2) et un virus de l'immunodéficience simienne (VIS), et ladite cellule cible du rétrovirus étant un lymphocyte T CD4+, une cellule dendritique, un monocyte ou un macrophage.

10. Cellule n'exprimant pas naturellement la protéine SAMHD1 dans laquelle la séquence codant la protéine SAMHD1 a été introduite selon la revendication 9, **caractérisée en ce que** ladite cellule provient d'un sujet et que son utilisation comme médicament pour traiter ou prévenir une maladie causée par un rétrovirus est réalisée chez ledit sujet, ledit rétrovirus étant un lentivirus sélectionné dans le groupe constitué par un virus de l'immunodéficience humaine de type 1 (VIH-1), un virus de l'immunodéficience humaine de type 2 (VIH-2) et un virus de l'immunodéficience simienne (VIS).

11. Méthode *in vitro* ou *ex vivo* selon la revendication 8, ou cellule n'exprimant pas naturellement la protéine SAMHD1 dans laquelle la séquence codant la protéine SAMHD1 a été introduite, selon la revendication 9 ou 10, **caractérisée en ce que** ladite cellule n'exprimant pas naturellement la protéine SAMHD1 est une cellule souche CD34+ capable de se différencier en lymphocyte T CD4+.

12. Méthode *in vitro* ou *ex vivo* selon la revendication 8 ou 11, ou cellule n'exprimant pas naturellement la protéine SAMHD1 dans laquelle la séquence codant la protéine SAMHD1 a été introduite, selon l'une quelconque des revendications 9 à 11, **caractérisée en ce que** l'introduction de la séquence du gène codant SAMHD1 dans ladite cellule n'exprimant pas naturellement la protéine SAMHD1 est réalisée à l'aide d'un vecteur de transfert de gène.

13. Une cellule n'exprimant pas naturellement la protéine SAMHD1, **caractérisée en ce qu'**elle exprime une protéine SAMHD1 recombinante, la séquence codant la protéine SAMHD1 recombinante ayant été introduite à l'aide d'un vecteur rétroviral comprenant cette séquence, ladite cellule étant une cellule souche hématopoïétique capable de se différencier i) en une cellule souche lymphoïde qui, à son tour, se divisera pour donner une cellule lymphoïde, et ii) en une cellule souche myéloïde qui, à son tour, se divisera pour donner une cellule de la lignée myéloïde.

## Patentansprüche

1. In-vitro-Verfahren zum Screenen von Kandidatenverbindungen, die geeignet sind, um für die präventive und/oder kurative Behandlung einer durch ein Retrovirus verursachten Krankheit verwendet zu werden, aufweisend die folgenden Schritte:
- in-Kontakt-Bringen einer Kandidatenverbindung mit einer SAMHD1 exprimierenden Zelle oder mit dem SAMHD1-Protein, und
- Ermitteln, ob die Kandidatenverbindung die Expression und/oder die Aktivität des SAMHD1-Proteins moduliert,
wobei die Kandidatenverbindung, die die Expression und/oder die Aktivität des SAMHD1-Proteins moduliert, als Kandidatenverbindung identifiziert wird, die geeignet ist, für die präventive und/oder kurative Behandlung einer durch ein Retrovirus verursachten Krankheit verwendet zu werden,
wobei das Retrovirus zur Gattung Lentivirus gehört und aus der Gruppe ausgewählt ist, die aus Arten besteht, die aus Humanen Immundefizienz-Viren des Typs 1 (HIV-1), Humanen Immundefizienz-Viren des Typs 2 (HIV-2) und Simianen Immundefizienz-Viren (SIV) bestehen.

2. In-vitro-Verfahren zum Screenen gemäß Anspruch 1, **dadurch gekennzeichnet, dass**:
- das Ermitteln der Fähigkeit der Kandidatenverbindung zum Modulieren der Expression von SAMHD1 durch Quantifizieren der SAMHD1-mRNA und/oder des SAMHD1-Proteins erfolgt, und
- das Ermitteln der Fähigkeit der Kandidatenverbindung zum Modulieren der Aktivität von SAMHD1 durch Messen der Phosphohydrolase-Aktivität von SAMHD1 erfolgt.

3. Inhibitor der Expression und/oder der Aktivität von SAMHD1 für dessen Verwendung bei der Behandlung und/oder Prävention einer durch ein Retrovirus verursachten Krankheit, wobei das Retrovirus ein Lentivirus ist, das aus der Gruppe bestehend aus Arten ausgewählt ist, die aus Humanen Immundefizienz-Viren des Typs 1 (HIV-1), Humanen Immundefizienz-Viren des Typs 2 (HIV-2) und Simianen Immundefizienz-Viren (SIV) bestehen, und wobei der Inhibitor aus der Gruppe bestehend aus einer interferierenden RNA, die gegen die mRNA oder die prä-mRNA von SAMHD1 gerichtet ist, einem Antisense-Oligonukleotid, das das SAMHD1 codierende Gen anspricht, einem Ribozym, das gegen die mRNA oder die prä-mRNA von SAMHD1 gerichtet ist, einem anti-SAMHD1-Antikörper, einem anti-SAMHD1-Antikörperderivat und einem Aptamer ausgewählt ist.

4. Immunogene oder vakzine Zusammensetzung, aufweisend:
i) einen Inhibitor der Expression und/oder der Aktivität des SAMHD1-Proteins, ausgewählt aus der Gruppe bestehend aus anti-SAMHD1-Antikörpern, interferierenden RNAs, die gegen die mRNA oder die prä-mRNA von SAMHD1 gerichtet sind, Antisense-Oligonukleotiden, die das SAMHD1 codierende Gen ansprechen, Ribozymen, die gegen die mRNA oder die prä-mRNA von SAMHD1 gerichtet sind, und Aptameren, und
ii) mindestens ein Antigen eines Retrovirus oder eine Polynukleotidsequenz, die dieses Antigen codiert, wobei das Retrovirus ein Lentivirus ist, das ausgewählt ist aus der Gruppe bestehend aus Humanen Immundefizienz-Viren des Typs 1 (HIV-1), Humanen Immundefizienz-Viren des Typs 2 (HIV-2) und Simianen Immundefizienz-Viren (SIV), und wobei das Antigen aus der Gruppe bestehend aus Hüllen-Glycoproteinen, dem Capsidprotein und dem Matrixprotein ausgewählt ist.

5. Immunogene oder vakzine Zusammensetzung gemäß Anspruch 4, **dadurch gekennzeichnet, dass** das Antigen aus der Gruppe bestehend aus einem Protein des Retrovirus oder einem Fragment desselben, einem Expressionsvektor aufweisend die Polynukleotidsequenz, die ein Protein des Retrovirus codiert oder ein Fragment derselben, einem virusähnlichen Partikel, welches Strukturproteine des Retrovirus besitzt, einem Lipopeptid-Antigen, aufweisend Proteine oder Peptidfragmente des Retrovirus, dem Retrovirus in inaktiver Form oder in abgeschwächter Form und einem pseudotypisiertem viralen Vektor ausgewählt ist.

6. In-vitro- oder Ex-vivo-Verwendung eines Inhibitors der Expression und/oder der Aktivität des SAMHD1-Proteins, der aus der Gruppe bestehend aus anti-SAMHD1-Antikörpern, interferierenden RNAs, die gegen die mRNA oder die prä-mRNA von SAMHD1 gerichtet sind, Antisense-Oligonukleotiden, die das SAMHD1 codierende Gen ansprechen, Ribozymen, die gegen die mRNA oder die prä-mRNA von SAMHD1 gerichtet sind, und Aptameren ausgewählt ist, um die Transduktion von Zellen, die SAMHD1 exprimieren, durch einen retroviralen Vektor zu erhöhen, wobei der retrovirale Vektor ein Humanes Immundefizienz-Virus des Typs 1 (HIV-1), ein Humanes Immundefizienz-Virus des Typs 2 (HIV-2) oder ein Simianes Immundefizienz-Virus (SIV) ist.

7. Kit zum Transduzieren eines retroviralen Vektors in eine Zelle, die SAMHD1 natürlich exprimiert, aufweisend:
- einen Inhibitor der Expression und/oder der Aktivität des SAMHD1-Proteins, der aus der Gruppe bestehend aus anti-SAMHD1-Antikörpern, interferierenden RNAs, die gegen die mRNA oder die prä-mRNA von SAMHD1 gerichtet sind, Antisense-Oligonukleotiden, die das SAMHD1 codierende Gen ansprechen, Ribozymen, die gegen die mRNA oder die prä-mRNA von SAMHD1 gerichtet sind, und Aptameren ausgewählt ist,
- einen retroviralen Vektor, der das Protein Vpx nicht natürlich exprimiert,
wobei der retrovirale Vektor ein lentiviraler Vektor ist, der ausgewählt ist aus der Gruppe bestehend aus einem Humanen Immundefizienz-Virus des Typs 1 (HIV-1), einem Humanen Immundefizienz-Virus des Typs 2 (HIV-2) und einem Simianen Immundefizienz-Virus (SIV).

8. In-vitro- oder Ex-vivo-Verfahren zum Induzieren einer Resistenz gegen die Infizierung durch ein Retrovirus in einer Zelle, die das SAMHD1-Protein nicht natürlich exprimiert, wobei das Verfahren aufweist eine Schritt bestehend aus dem Einführen einer Sequenz, die das SAMHD1-Protein codiert, die die Zelle, die das SAMHD1-Protein nicht natürlich exprimiert, wobei die Zellen, die das SAMHD1-Protein exprimieren, gegen die Infektion durch ein Retrovirus resistent sind, wobei das Retrovirus ein Lentivirus ist, das ausgewählt ist aus der Gruppe bestehend aus einem Humanen Immundefizienz-Virus des Typs 1 (HIV-1), einem Humanen Immundefizienz-Virus des Typs 2 (HIV-2) und einem Simianen Immundefizienz-Virus (SIV).

9. Eine Zelle, die das SAMHD1-Protein nicht natürlich exprimiert, in die die Sequenz, die das SAMHD1-Protein codiert, eingeführt wurde, wobei die Zelle eine Zielzelle eines Retrovirus bei einer Infektion oder eine Zelle ist, die in der Lage ist, sich in eine Zielzelle des Retrovirus auszudifferenzieren, für ihre Verwendung als Medikament zum Behandeln oder Vorbeugen einer Krankheit, die durch ein Retrovirus verursacht wird, wobei das Retrovirus ein Lentivirus ist, das ausgewählt ist aus der Gruppe bestehend aus einem Humanen Immundefizienz-Virus des Typs 1 (HIV-1), einem Humanen Immundefizienz-Virus des Typs 2 (HIV-2) und einem Simianen Immundefizienz-Virus (SIV), und wobei die Zielzelle des Retrovirus ein CD4+-T-Lymphozyt, eine dendritische Zelle, ein Monozyt oder ein Makrophage ist.

10. Zelle, die das SAMHD1-Protein nicht natürlich exprimiert, in die die Sequenz, die das SAMHD1-Protein codiert, eingeführt wurde, gemäß Anspruch 9, **dadurch gekennzeichnet, dass** die Zelle von einer Person stammt und ihre Verwendung als Medikament zum Behandeln oder Vorbeugen einer durch ein Retrovirus verursachten Krankheit bei der Person realisiert wird, wobei das Retrovirus ein Lentivirus ist, das ausgewählt ist aus der Gruppe bestehend aus einem Humanen Immundefizienz-Virus des Typs 1 (HIV-1), einem Humanen Immundefizienz-Virus des Typs 2 (HIV-2) und einem Simianen Immundefizienz-Virus (SIV).

11. In-vitro- oder Ex-vivo-Verfahren gemäß Anspruch 8 oder Zelle, die das SAMHD1-Protein nicht natürlich exprimiert, in die die Sequenz, die das SAMHD1-Protein codiert, eingeführt wurde, gemäß Anspruch 9 oder 10, **dadurch gekennzeichnet, dass** die Zelle, die das SAMHD1-Protein nicht natürlich exprimiert, eine CD34+-Stammzelle ist, die in der Lage ist, sich in einen CD4+-T-Lymphozyten auszudifferenzieren.

12. In-vitro- oder Ex-vivo-Verfahren gemäß Anspruch 8 oder 11, oder Zelle, die das SAMHD1-Protein nicht natürlich exprimiert, in die die Sequenz, die das SAMHD1-Protein codiert, eingeführt wurde, gemäß irgendeinem der Ansprüche 9 bis 11, **dadurch gekennzeichnet, dass** das Einführen der Sequenz des SAMHD1 codierenden Gens in die Zelle, die das SAMHD1-Protein nicht natürlich exprimiert, mit Hilfe eines Gentransfervektors realisiert wird.

13. Eine Zelle, die das SAMHD1-Protein nicht natürlich exprimiert, **dadurch gekennzeichnet, dass** sie ein rekombinantes SAMHD1-Protein exprimiert, wobei die Sequenz, die das rekombinante SAMHD1-Protein codiert, mit Hilfe eines retroviralen Vektors eingeführt wurde, der diese Sequenz aufweist, wobei die Zelle eine hämatopoetische Stammzelle ist, die in der Lage ist, sich i) in eine lymphatische Stammzelle, die sich ihrerseits teilt, um eine lymphatische Zelle zu ergeben, und ii) in eine myeloische Zelle, die sich ihrerseits teilt, um eine Zelle der myeloischen Linie zu ergeben, auszudifferenzieren.

## Claims

1. An *in vitro* method of screening candidate compounds capable of being used for the preventive and/or curative treatment of a disease caused by a retrovirus, comprising the following steps:
- contacting a candidate compound with a cell expressing SAMHD1 or with the SAMHD1 protein; and
- determining whether the candidate compound modulates the expression and/or the activity of the SAMHD1 protein;
wherein said candidate compound which modulates the expression and/or the activity of the SAMHD1 protein is identified as a candidate compound capable of being used for the preventive and/or curative treatment of a disease caused by a retrovirus,
wherein said retrovirus belongs to the lentivirus genus and is selected from the group consisting of species consisting of human immunodeficiency viruses type 1 (HIV-1), human immunodeficiency viruses type 2 (HIV-2) and simian immunodeficiency viruses (SIV).

2. An *in vitro* method of screening according to claim 1, **characterised in that**:
- the determination of the ability of the candidate compound to modulate the expression of SAMHD1 is carried out by quantifying the SAMHD1 mRNA and/or the SAMHD1 protein ; and
- the determination of the ability of the candidate compound to modulate the activity of SAMHD1 is carried out by measuring the phosphohydrolase activity of SAMHD1.

3. An inhibitor of the expression and/or the activity of SAMHD1 for use in the treatment and/or prevention of a disease caused by a retrovirus, wherein said retrovirus is a lentivirus selected from the group consisting of species consisting of human immunodeficiency viruses type 1 (HIV-1), human immunodeficiency viruses type 2 (HIV-2) and simian immunodeficiency viruses (SIV), and wherein said inhibitor is selected from the group consisting of an interfering RNA directed against the SAMHD1 mRNA or pre-mRNA, an antisense oligonucleotide targeting the gene encoding SAMHD1, a ribozyme directed against the SAMHD1 mRNA or pre-mRNA, an anti-SAMHD1 antibody, or an anti-SAMHD1 antibody derivative, and an aptamer.

4. An immunogenic or vaccine composition comprising:
i) an inhibitor of the expression and/or activity of the SAMHD1 protein selected from the group consisting of anti-SAMHD1 antibodies, interfering RNAs directed against the SAMHD1 mRNA or pre-mRNA, antisense oligonucleotides targeting the gene encoding SAMHD1, ribozymes directed against the SAMHD1 mRNA or pre-mRNA and aptamers ; and
ii) at least one antigen of a retrovirus or a polynucleotide sequence encoding this antigen, wherein said retrovirus is a lentivirus selected from the group consisting of human immunodeficiency viruses type 1 (HIV-1), human immunodeficiency viruses type 2 (HIV-2) and simian immunodeficiency viruses (SIV), and wherein said antigen is selected from the group consisting of envelope glycoproteins, capsid protein, and matrix protein.

5. An immunogenic or vaccine composition according to claim 4, **characterised in that** the antigen is selected from the group consisting of : a protein of said retrovirus or a fragment thereof, an expression vector comprising the polynucleotide sequence encoding a protein of said retrovirus or a fragment thereof, a virus like particle having the structural proteins of said retrovirus, a lipopeptide antigen comprising proteins or peptide fragments of said retrovirus, said retrovirus in its inactivated form or in its attenuated form, and a pseudotyped viral vector.

6. *In vitro* or *ex vivo* use of an inhibitor of the expression and/or the activity of the SAMHD1 protein selected from the group consisting of anti-SAMHD1 antibodies, interfering RNAs directed against the SAMHD1 mRNA or pre-mRNA, antisense oligonucleotides targeting the gene encoding SAMHD1, ribozymes directed against the SAMHD1 mRNA or pre-mRNA and aptamers in order to increase the transduction of cells expressing SAMHD1 by a retroviral vector, wherein said retroviral vector is a human immunodeficiency virus type 1 (HIV-1), a human immunodeficiency virus type 2 (HIV-2) or a simian immunodeficiency virus (SIV).

7. A kit for transducing a retroviral vector in a cell naturally expressing SAMHD1, comprising:
- an inhibitor of the expression and/or the activity of the SAMHD1 protein selected from the group consisting of anti-SAMHD1 antibodies, interfering RNAs directed against the SAMHD1 mRNA or pre-mRNA, antisense oligonucleotides targeting the gene encoding SAMHD1, ribozymes directed against the SAMHD1 mRNA or pre-mRNA and aptamers ;
- a retroviral expression vector that does not naturally express the Vpx protein, said retroviral vector being a lentiviral vector selected from the group consisting of a human immunodeficiency virus type 1 (HIV-1), a human immunodeficiency virus type 2 (HIV-2) and a simian immunodeficiency virus (SIV).

8. An *in vitro* or *ex vivo* method for inducing resistance to infection by a retrovirus, in a cell that does not naturally express the SAMHD1 protein, said method comprising a step consisting of introducing a sequence encoding the SAMHD1 protein in the said cell that does not naturally express the SAMHD1 protein, the cells expressing the SAMHD1 protein being resistant to infection by a retrovirus, wherein said retrovirus is a lentivirus selected from the group consisting of a human immunodeficiency virus type 1 (HIV-1), a human immunodeficiency virus type 2 (HIV-2) and a simian immunodeficiency virus (SIV).

9. A cell not naturally expressing the SAMHD1 protein in which the sequence encoding the SAMHD1 protein has been introduced, said cell being a target cell of the retrovirus during infection or a cell that is capable of differentiating into a target cell of the retrovirus, for use thereof as a medicament for treating or preventing a disease caused by a retrovirus, wherein said retrovirus is a lentivirus selected from the group consisting of a human immunodeficiency virus type 1 (HIV-1), a human immunodeficiency virus type 2 (HIV-2) and a simian immunodeficiency virus (SIV), and wherein said target cell of the retrovirus is a CD4+ T lymphocyte, a dendritic cell, a monocyte or a macrophage.

10. A cell not naturally expressing the SAMHD1 protein in which the sequence encoding the SAMHD1 protein has been introduced according to claim 9, **characterised in that** said cell is derived from a subject and **in that** its use as a medicament for treating or preventing a disease caused by a retrovirus is performed in said subject, wherein said retrovirus is a lentivirus selected from the group consisting of a human immunodeficiency virus type 1 (HIV-1), a human immunodeficiency virus type 2 (HIV-2) and a simian immunodeficiency virus (SIV) .

11. An *in vitro* or *ex vivo* method according to claim 8, or a cell that does not naturally express the SAMHD1 protein wherein the sequence encoding the SAMHD1 protein has been introduced, according to claim 9 or 10, **characterised in that** said cell not naturally expressing the SAMHD1 protein is a CD34+ stem cell capable of differentiating into a CD4+ T lymphocyte.

12. An *in vitro* or *ex vivo* method according to claim 8 or 11, or a cell that does not naturally express the SAMHD1 protein wherein the sequence encoding the SAMHD1 protein has been introduced, according to any one of claims 9 to 11, **characterised in that** the introduction of the sequence of the gene encoding SAMHD1 in said cell not naturally expressing the SAMHD1 protein is carried out by using a gene transfer vector.

13. A cell that does not naturally express the SAMHD1 protein, **characterised in that** it expresses a recombinant SAMHD1 protein, wherein the sequence encoding the recombinant SAMHD1 protein has been introduced by a retroviral vector comprising said sequence, wherein said cell is a hematopoietic stem cell capable of differentiating i) into lymphoid stem cells which would in turn divide to give lymphoid cells, and ii) into myeloid stem cells which would in turn divide to give cells of the myeloid cell lineage.


### SEQUENCE LISTING

<110> CENTRE NATIONAL DE LA RECHERCHE SCIENTIFIQUE
<120> METHODE DE CRIBLAGE DE COMPOSES ANTIRETROVIRAUX ET VACCIN
<130> BET 12P0412
<160> 6
<170> PatentIn version 3.5
<210> 1
   <211> 626
   <212> PRT
   <213> Homo sapiens
<400> 1
<210> 2
   <211> 3189
   <212> DNA
   <213> Homo sapiens
<220>
   <221> misc_feature
   <222> (201)..(2081)
   <223> CDS
<400> 2
   attgcgcctg cgcagggagc ccaaggcaag agccgctagg ctgccctgcc cgaagggctc      60
   aactgtcagt gagcctgcgc aggaggccaa taggctgcca atactccttg gactccccgc     120
   cagggccctg ctgtcagtgc gcctgcgcgc gggtccggcg ccgaggttct tgactgctgt     180
   gccggacgcc aggtgtagcc atgcagcgag ccgattccga gcagccctcc aagcgtcccc     240
   gttgcgatga cagcccgaga accccctcaa acaccccttc cgcagaggca gactggtccc     300
   cgggcctgga actccatccc gactacaaga catggggtcc ggagcaggtg tgctccttcc     360
   tcaggcgcgg tggctttgaa gagccggtgc tgctgaagaa catccgagaa aatgaaatca     420
   caggcgcatt actgccttgt cttgatgagt ctcgttttga aaatcttgga gtaagttcct     480
   tgggggagag gaagaagctg cttagttata tccagcgatt ggttcaaatc cacgttgata     540
   caatgaaggt aattaatgat cctatccatg gccacattga gctccaccct ctcctcgtcc     600
   gaatcattga tacacctcaa tttcaacgtc ttcgatacat caaacagctg ggaggtggtt     660
   actatgtttt tccaggagct tcacacaatc gatttgagca tagtctaggg gtggggtatc     720
   tagcaggatg tctagttcac gcactgggtg aaaaacaacc agagctgcag ataagtgaac     780
   gagatgttct ctgtgttcag attgctggac tttgtcatga tctcggtcat gggccatttt     840
   ctcacatgtt tgatggacga tttattccac ttgctcgccc ggaggtgaaa tggacgcatg     900
   aacaaggctc agttatgatg tttgagcacc ttattaattc taatggaatt aagcctgtca     960
   tggaacaata tggtctcatc cctgaagaag atatttgctt tataaaggaa caaattgtag    1020
   gaccacttga atcacctgtc gaagattcat tgtggccata taaagggcgt cctgaaaaca    1080
   aaagcttcct ttatgagata gtatctaata aaagaaatgg cattgatgtg gacaaatggg    1140
   attattttgc cagggactgc catcatcttg gaatccaaaa taattttgat tacaagcgct    1200
   ttattaagtt tgcccgtgtc tgtgaagtag acaatgagtt gcgtatttgt gctagagata    1260
   aggaagttgg aaatctgtat gacatgttcc acactcgcaa ctctttacac cgtagagctt    1320
   atcaacacaa agttggcaac attattgata caatgattac agatgctttc ctcaaagcag    1380
   atgactacat agagattaca ggtgctggag gaaaaaagta tcgcatttct acagcaattg    1440
   acgacatgga agcctatact aagctgacag ataacatttt tctggagatt ttatactcta    1500
   ctgatcccaa attgaaagac gcacgagaga ttttaaaaca aattgaatac cgtaatctat    1560
   tcaagtatgt gggtgagacg cagccaacag gacaaataaa gattaaaagg gaggactatg    1620
   aatctcttcc aaaagaggtt gccagtgcta aacccaaagt attgctagac gtgaaactga    1680
   aggctgaaga ttttatagtg gatgttatca acatggatta tggaatgcaa gaaaagaatc    1740
   caattgatca tgttagcttc tattgtaaga ctgcccccaa cagagcaatc aggattacta    1800
   aaaaccaggt ttcacaactt ctgccagaga aatttgcaga gcagctgatt cgagtatatt    1860
   gtaagaaggt ggacagaaag agtttgtatg ccgcaagaca atattttgtt cagtggtgtg    1920
   cagacagaaa tttcaccaag ccgcaggatg gcgatgttat agccccactc ataacacctc    1980
   aaaaaaagga atggaacgac agtacttcag tccaaaatcc aactcgcctc cgagaagcat    2040
   ccaaaagcag agtccagctt tttaaagatg acccaatgtg aatgtctgta gtcagttgtt    2100
   tacaaactcc ctctcctgca caattcattt agaggcttca atcatagaat tctgcaaatt    2160
   aatgacaact catgctttaa ttttgtattt tgaatgtaca cgcatgctga agctaagtaa    2220
   cttttaatca aagaaataag atggtattag gcaaatctta ctatactatg aaaagcatta    2280
   ccttgcctat ttttaatatt attaaagcct ttctccttca gtagtctatt ttcttagaat    2340
   aacaactctt ttatctattc tgaactctat tttttttctt ttttaagaga caaggttttg    2400
   ctctgttgcc cagcttggac tcgaactttc ctgggctcaa gcgaccctcc tgcctcagcc    2460
   ccccaagtag ctgggactaa agtcatgtgc caccacaccc agcttactct gaacttttat    2520
   gacagatgat tgttttttgt ttttaatgta gaaatgagac aagggtacaa attggaacta    2580
   gaaattgaca ttgtcattga caaacatggc taaaaacaaa acatcaaatc ctgcccccgt    2640
   gaagagttcc ctgtcacctc aagtttgagg atagtcctct aagagtgacc taagcataag    2700
   tgaaagacac ctcccctcac ccttctagcc ccctacaagg tgccaggttg gggtaaagag    2760
   ttggagatga tggccaggag tggcctccaa cacgctggtg agaggcctga ttaggttttg    2820
   gggaagatct gagagctctg gcctcttcgt gagtggaaca taaagccgcc tcttgttggg    2880
   agatcctacc ccagtgacag aggaatcccc caaactaggc tgtgccctgg ctccgtggcg    2940
   gctccagacc cgggtagtgc ctttgtcccc tgaatactca ctcccccggt ccagagggcc    3000
   ttcccactgc ccagcctgga gaaggcctcc cctgacctgc tctctcagta tcctggagag    3060
   ctggccagag gccatcacag gcatcatcct cagagctcct cagacctggg actttgtttt    3120
   tgctggttca gtgcattttg tgtatttaag agcaaacact agccaggcgt ggcggcgtgt    3180
   gcctgtagt                                                            3189
<210> 3
   <211> 19
   <212> RNA
   <213> Artificial Sequence
<220>
   <223> samhd1 siRNA
<400> 3
   gauucauugu ggccauaua 19
<210> 4
   <211> 19
   <212> RNA
   <213> Artificial Sequence
<220>
   <223> samhd1 siRNA
<400> 4
   caaccagagc ugcagauaa 19
<210> 5
   <211> 19
   <212> RNA
   <213> Artificial Sequence
<220>
   <223> samhd1 shRNA (sens)
<400> 5
   cgcaggaugg cgauguuau 19
<210> 6
   <211> 19
   <212> RNA
   <213> Artificial Sequence
<220>
   <223> samhd1 shRNA (antisens)
<400> 6
   auaacaucgc cauccugcg 19